# EUROPÄISCHE PATENTSCHRIFT

(11) **EP 3 528 854 B1**
(45) Veröffentlichungstag und Bekanntmachung des Hinweises auf die Patenterteilung: **14.08.2024**
(21) Anmeldenummer: 17835454.4
(22) Anmeldetag: 21.10.2017
(51) Int. Cl.: A61L 9/12, A61M 11/04, A61M 15/00, A61M 15/06

(54) **VORRICHTUNG ZUM ABDUNSTEN VON WIRKSTOFFEN**
DEVICE FOR EVAPORATING ACTIVE SUBSTANCES
DISPOSITIF DE DÉGAGEMENT DE SUBSTANCES ACTIVES

(30) Priorität: 21.10.2016 DE 102016120145
(43) Veröffentlichungstag der Anmeldung: 28.08.2019
(73) Patentinhaber: Arrows Biomedical Products GmbH, 33102 Paderborn (DE)
(72) Erfinder: RAEM, Arnold M., 48159 Münster (DE)
(74) Vertreter: Werner & ten Brink
(86) Internationale Anmeldenummer: PCT/EP2017/076930
(87) Internationale Veröffentlichungsnummer: WO 2018/073450

(56) Entgegenhaltungen:
- EP-A1- 0 316 266
- WO-A1-80/00003
- JP-A- H08 196 612
- US-B1- 8 765 063

## Beschreibung

Die vorliegende Erfindung bezieht sich auf eine Vorrichtung zum Abdunsten von Wirkstoffen, umfassend einen Behälter für den Wirkstoff und eine Verdunsteinheit, an der ein Kappenelement angeordnet ist, das relativ zur Verdunsteinheit in eine geöffnete und in eine geschlossene Position verschiebbar ist, wobei die Verdunsteinheit in der geschlossenen Position durch das Kappenelement abgedeckt ist und in der geöffneten Position zumindest teilweise freiliegt.

Derartige Vorrichtungen werden zur passiven oder aktiven Abdunstung des Wirkstoffs, der z. B. ein Parfum, ein ätherisches Öl oder ein paramedizinischer Wirkstoff, beispielsweise gegen Erkältungsbeschwerden oder zum Entwöhnen des Rauchens, sein kann, oder in ähnlicher Weise verwendet. Für die aktive Abdunstung wird das Kappenelement in die geöffnete Position gebracht und die Vorrichtung in die Nähe von Mund und/oder Nase gehalten, um den Wirkstoff zu inhalieren. Aus der US 4,938,419 bzw. der EP 316 266 A1 ist beispielsweise eine Vorrichtung zur Abdunstung von Wirkstoffen bekannt, bei der an einem Hohlkörper, der als Verdunstelement dient, umlaufend eine sich nach außen erweiternden Einschubdichtungsnut angeordnet ist, um die Vorrichtung gegen Wirkstoffverlust abzudichten. Ein Abdeckorgan weist zum Eingriff in die Einschubdichtungsnut bei geschlossener Position in einem Randabschnitt des Abdeckorgans eine zur Einschubdichtungsnut gegengleiche Verjüngung auf. Die im Bereich der Einschubdichtungsnut in geschlossener Position aufeinanderliegenden Flächen des Hohlkörpers und des Abdeckorgans sind außerdem als dicht aufeinanderliegende Dichtflächen ausgebildet. Außerdem ist zur Begrenzung der maximalen Auszugsposition des Abdeckorgans ein mittig angeordneter zylindrischer Anschlagnocken vorgesehen, der an einer Innenseite des Abdeckorgans in eine Anschlaglängsnut des Hohlkörpers eingreift und an einer Außenseite des Abdeckorgans bündig mit dessen Oberfläche abschließt. Die Anschlaglängsnut wird beidseitig durch Stege begrenzt.

Aus der JP H08-196612 A ist eine Aufnahmevorrichtung für flüchtig chemische Mittel bekannt, die so ausgebildet ist, dass ein chemisches Gel in einem Chemikalienbehälter aus Behälterkörper und Abdeckkörper untergebracht ist. Die Chemikalie kann sich durch Öffnungen im Behälter nach außen verflüchtigen. Wenn die Gehäusevorrichtung umgedreht wird, wird die Luftkontaktfläche des Gels geändert.

Die WO 80/00003 A1 offenbart ein Organ zur Langzeitabdunstung, bei dem ein Grundkörper mit einer Duftstoffreservekammer versehen ist, die mittels

Duftdurchtrittkanälen mit einer Abdunstungskammer kommuniziert, und mit einem in wenigstens zwei Positionen verschiebbaren Abdeckkörper.

Es ist eine Aufgabe der vorliegenden Erfindung, eine Vorrichtung zum Abdunsten von Wirkstoffen mit einer verbesserten Technik zum Öffnen und Schließen anzugeben.

Diese Aufgabe wird gemäß Anspruch 1 dadurch gelöst, dass bei einer Vorrichtung zum Abdunsten von Wirkstoffen, umfassend einen Behälter für den Wirkstoff und eine Verdunsteinheit, an der ein Kappenelement angeordnet ist, das relativ zur Verdunsteinheit in eine geöffnete und in eine geschlossene Position verschiebbar ist, wobei die Verdunsteinheit in der geschlossenen Position durch das Kappenelement abgedeckt ist und in der geöffneten Position zumindest teilweise freiliegt, eine Verschlussmechanik, umfassend ein erstes Verschlussteil, das an der Verdunsteinheit angeordnet ist, und ein zweites, zum ersten Verschlussteil komplementäres Verschlussteil, das an einer Innenseite des Kappenelements angeordnet ist, vorgesehen ist, wobei die Verschlussteile derart zusammenwirken, dass sich das erste oder das zweite Verschlussteil bei einer vollständig geöffneten Position des Kappenelements in einer Anschlagposition befindet und beim Bewegen zwischen geöffneter und geschlossener Position des Kappenelements das erste Verschlussteil die Innenseite des Kappenelements oder das zweite Verschlussteil die Verdunsteinheit parallel zur Verschiebungsrichtung kontaktiert. Dabei ist das erste Verschlussteil als Rasthaken ausgebildet, der an einer dem Behälter für Wirkstoff abgewandten Seite der Verdunsteinheit angeordnet ist, weist das Kappenelement an der Innenseite eine als Nut ausgebildete Vertiefung auf, in der der Rasthaken beim Bewegen zwischen geöffneter und geschlossener Position des Kappenelements gleitet, und ist an der Innenseite des Kappenelements ein Anschlagelement für den Rasthaken angeordnet, an dem der Rasthaken in einer vollständig geöffneten Position des Kappenelements anschlägt.

Ein Vorteil der Erfindung besteht darin, dass das Kappenelement beim Bewegen zwischen geöffneter und geschlossener Position durch den stetigen Kontakt der Verschlussteile mit der Innenseite des Kappenelements oder der Verdunsteinheit besonders stabil geführt wird, wodurch eine angenehme Haptik bei der Benutzung der Vorrichtung entsteht. Auf diese vorgeschlagene Weise entsteht eine sicherere Verschlussmechanik ohne Kleinteile, die beim Benutzen der Vorrichtung zugänglich sind und abbrechen oder versehentlich herausfallen können. Somit besteht, insbesondere für Kinder, keine Gefährdung durch Einatmen derartiger Kleinteile. Es ergibt sich insgesamt ein fester Verschluss der Vorrichtung, der sich dennoch leicht öffnen lässt.

In bevorzugten Ausführungsformen weisen die Verdunsteinheit und das Kappenelement je eine zur Verschiebungsrichtung im Wesentlichen parallele Seitenfläche auf, ist der Rasthaken an der Seitenfläche der Verdunsteinheit angeordnet, und sind die Vertiefung und das Anschlagelement an der Seitenfläche des Kappenelements angeordnet. Auf diese Weise gleitet der Rasthaken besonders gleichmäßig in der Vertiefung.

An der Verdunsteinheit ist vorzugsweise an mindestens einer der Innenseite des Kappenelements zugewandten Seite ein Führungselement angeordnet. Dadurch kann das Kappenelement beim Bewegen zwischen geöffneter und geschlossener Position noch stabiler geführt werden.

In einem weiteren Aspekt wird die Aufgabe gemäß Anspruch 4 dadurch gelöst, dass bei einer Vorrichtung zum Abdunsten von Wirkstoffen, umfassend einen Behälter für den Wirkstoff und eine Verdunsteinheit, an der ein Kappenelement angeordnet ist, das relativ zur Verdunsteinheit in eine geöffnete und in eine geschlossene Position verschiebbar ist, wobei die Verdunsteinheit in der geschlossenen Position durch das Kappenelement abgedeckt ist und in der geöffneten Position zumindest teilweise freiliegt, eine Verschlussmechanik, umfassend ein erstes Verschlussteil, das an der Verdunsteinheit angeordnet ist, und ein zweites, zum ersten Verschlussteil komplementäres Verschlussteil, das an einer Innenseite des Kappenelements angeordnet ist, vorgesehen ist, wobei die Verschlussteile derart zusammenwirken, dass sich das erste oder das zweite Verschlussteil bei einer vollständig geöffneten Position des Kappenelements in einer Anschlagposition befindet und beim Bewegen zwischen geöffneter und geschlossener Position des Kappenelements das erste Verschlussteil die Innenseite des Kappenelements oder das zweite Verschlussteil die Verdunsteinheit parallel zur Verschiebungsrichtung kontaktiert. Dabei ist das erste Verschlussteil als Rastnase ausgebildet, die an einer dem Behälter für Wirkstoff abgewandten Seite der Verdunsteinheit angeordnet ist, und weist das Kappenelement an der Innenseite eine Vertiefung für die Rastnase auf, in die die Rastnase in einer vollständig geöffneten Position des Kappenelements einrastet, und kontaktiert die Rastnase beim Bewegen zwischen geöffneter und geschlossener Position und in der geschlossenen Position des Kappenelements die Innenseite des Kappenelements parallel zur Verschiebungsrichtung.

Dadurch ergibt sich eine sicherere Verschlussmechanik. Die Rastnase kann in der vollständig geöffneten Position des Kappenelements in die Vertiefung einrasten. Beim Bewegen des Kappenelements wird die Rastnase beispielsweise eingedrückt und kontaktiert so die Innenseite des Kappenelements bzw. die Verdunsteinheit. Die Vorrichtung weist somit keine Kleinteile auf, die beim Benutzen der Vorrichtung zugänglich sind und abbrechen oder versehentlich herausfallen können. Somit besteht, insbesondere für Kinder, keine Gefährdung durch Einatmen derartiger Kleinteile. Insgesamt weisen solche Vorrichtungen einen festen Verschluss auf, der sich dennoch leicht öffnen lässt.

Bevorzugt weisen die Verdunsteinheit und das Kappenelement je zwei zur Verschiebungsrichtung im Wesentlichen parallele Seitenflächen auf, und sind genau zwei Rastnasen an den Seitenflächen der Verdunsteinheit angeordnet, und sind genau zwei Vertiefungen an den Seitenflächen des Kappenelements angeordnet. Dadurch entsteht eine noch stabilere Führung beim Bewegen des Kappenelements, bei dem die beiden Rastnasen die Seitenflächen des Kappenelements gleichmäßig kontaktieren.

In einem weiteren Aspekt wird die Aufgabe gemäß Anspruch 6 dadurch gelöst, dass bei einer Vorrichtung zum Abdunsten von Wirkstoffen, umfassend einen Behälter für den Wirkstoff und eine Verdunsteinheit, an der ein Kappenelement angeordnet ist, das relativ zur Verdunsteinheit in eine geöffnete und in eine geschlossene Position verschiebbar ist, wobei die Verdunsteinheit in der geschlossenen Position durch das Kappenelement abgedeckt ist und in der geöffneten Position zumindest teilweise freiliegt, eine Verschlussmechanik, umfassend ein erstes Verschlussteil, das an der Verdunsteinheit angeordnet ist, und ein zweites, zum ersten Verschlussteil komplementäres Verschlussteil, das an einer Innenseite des Kappenelements angeordnet ist, vorgesehen ist, wobei die Verschlussteile derart zusammenwirken, dass sich das erste oder das zweite Verschlussteil bei einer vollständig geöffneten Position des Kappenelements in einer Anschlagposition befindet und beim Bewegen zwischen geöffneter und geschlossener Position des Kappenelements das erste Verschlussteil die Innenseite des Kappenelements oder das zweite Verschlussteil die Verdunsteinheit parallel zur Verschiebungsrichtung kontaktiert. Dabei ist das erste Verschlussteil als Vorsprung ausgebildet, der an der Verdunsteinheit beabstandet zum Behälter für Wirkstoff angeordnet ist, und das Kappenelement an der Innenseite eine als Führungsrille ausgebildete Vertiefung aufweist, in der der Vorsprung beim Bewegen zwischen geöffneter und geschlossener Position des Kappenelements gleitet, und dass an der Innenseite des Kappenelements ein Anschlagelement für den Vorsprung angeordnet ist, an dem der Vorsprung in einer vollständig geöffneten Position des Kappenelements anschlägt.

Dadurch ergibt sich eine sicherere Verschlussmechanik. Beim Bewegen des Kappenelements kontaktiert der Vorsprung die Innenseite des Kappenelements bzw. die Verdunsteinheit. Die Vorrichtung weist somit keine Kleinteile auf, die beim Benutzen der Vorrichtung zugänglich sind und abbrechen oder versehentlich herausfallen können. Somit besteht, insbesondere für Kinder, keine Gefährdung durch Einatmen derartiger Kleinteile.Insgesamt lassen sich solche Vorrichtungen lassen sich besonders leicht öffnen, was insbesondere für eine einhändige Bedienung von Vorteil ist.

Vorteilhafterweise weisen die Verdunsteinheit und das Kappenelement je zwei zur Verschiebungsrichtung im Wesentlichen parallele Seitenflächen auf, sind genau zwei Vorsprünge an den Seitenflächen der Verdunsteinheit angeordnet und sind genau zwei Vertiefungen an den Seitenflächen des Kappenelements angeordnet. Dadurch entsteht eine noch stabilere Führung beim Bewegen des Kappenelements, bei dem die beiden Vorsprünge die Seitenflächen des Kappenelements gleichmäßig kontaktieren.

In bevorzugten Ausführungsformen ist außen an dem Kappenelement oder am Behälter für Wirkstoff ein Befestigungselement angeordnet. Auf diese Weise muss die Vorrichtung während oder nach der Benutzung nicht in der Hand gehalten werden, sondern kann beispielsweise an der Kleidung befestigt werden. In oder an Taschen ist ein sicherer Transport der Vorrichtung möglich, ein Herunterfallen der Vorrichtung wird vermieden.

Das Befestigungselement ist bevorzugt derart ausgeformt, dass es zumindest teilweise beabstandet von und parallel zu dem Behälter verläuft, falls das Befestigungselement am Kappenelement angeordnet ist. Falls es am Wirkstoffbehälter angeordnet ist, verläuft es zumindest teilweise beabstandet von und parallel zum Kappenelement. Dadurch kann die Vorrichtung so an einem Gegenstand befestigt werden, dass sich ein Teil des Gegenstands zwischen dem Befestigungselement und dem Behälter bzw. dem Kappenelement befindet. Der Gegenstand kann beispielsweise ein Kleidungsstück, z. B. eine Hemdtasche, oder eine andere Tasche sein, dessen Stoff oder Material zwischen den Behälter bzw. dem Kappenelement und den parallel dazu verlaufenden Teil des Befestigungselements geschoben wird. So kann ein versehentliches Heraus- oder Herunterfallen beim Transport der Vorrichtung vermieden werden. Durch Druck auf den beabstandet und parallel zum Behälter bzw. Kappenelement verlaufenden Teil des Befestigungselements gleichzeitig parallel zu und in Richtung des Behälters bzw. Kappenelements kann außerdem das Kappenelement verschoben werden, so dass die Vorrichtung einhändig bedient werden kann, also z. B. in einer Hand gehalten und mit einem Finger dieser Hand geöffnet und geschlossen werden kann.

Bevorzugt weist der Behälter bzw. das Kappenelement eine Führungsnut auf, je nachdem ob das Befestigungselement am Kappenelement oder am Behälter angeordnet ist. Dadurch ergibt sich eine Führung für das Befestigungselement beim Bewegen des Kappenelements zwischen der geöffneten und geschlossenen Position mittels des Befestigungselements, welches in oder entlang dieser Führungsnut gleiten kann, wodurch die Haptik beim Öffnen und Schließen der Vorrichtung verbessert ist.

Vorteilhafterweise ist der parallel zum Behälter bzw. Kappenelement verlaufende Teil des Befestigungselements zumindest teilweise drückbar. Drückbar bedeutet in diesem Zusammenhang, dass ein Teil des Befestigungselements gegen den Behälter bzw. das Kappenelement gedrückt werden kann, so dass dieser Teil den Behälter bzw. das Kappenelement kontaktiert. Der parallel zum Behälter bzw. Kappenelement verlaufende Teil des Befestigungselements kann beispielsweise gedrückt werden und mittels des Befestigungselements das Kappenelement verschoben werden. Dadurch entsteht eine verbesserte Haptik und stabilere Führung beim Verschieben des Kappenelements, und das einhändige Bedienen der Vorrichtung wird erleichtert.

In besonders vorteilhaften Ausführungsformen weist der Behälter sowohl eine Führungsnut als auch einen parallel zum Behälter bzw. Kappenelement verlaufenden Teil des Befestigungselements, der teilweise drückbar ist, auf. Dadurch ist der drückbare Teil in die Führungsnut drückbar, wodurch sich eine besonders gute Führung des Befestigungselements und damit des Kappenelements beim Bewegen zwischen der geöffneten und geschlossenen Position ergibt.

In bevorzugten Ausführungsformen weist das Befestigungselement an einem Ende eine Verdickung auf, die den Behälter kontaktiert. Die Verdickung kann gegebenenfalls in der Führungsnut gleiten, wenn das Kappenelement bewegt wird, so dass das Befestigungselement mittels der Verdickung seitlich geführt wird.

Bevorzugt weist das Kappenelement an der dem Behälter abgewandten Seite, insbesondere wenn am Kappenelement ein Befestigungselement angeordnet ist, oder der Behälter an der dem Kappenelement abgewandten Seite, insbesondere wenn am Behälter ein Befestigungselement angeordnet ist, abgerundete Ecken auf. Dies hilft dem Benutzer zu erkennen, an welcher Seite der Behälter und an welcher das Kappenelement ist.

Die Vorrichtung weist in vorteilhaften Ausführungsformen eine Vorderseite und eine Rückseite auf, die im Wesentlichen parallel zueinander verlaufen, wobei die Rückseite in einem Übergangsbereich zur Vorderseite eine abgerundete Form aufweist. Auf diese Weise kann der Benutzer die Vorderseite der Vorrichtung leichter von der Rückseite unterscheiden. Die Unterscheidung zwischen Vorder- und Rückseite ist für den Benutzer dadurch sowohl rein optisch, als auch rein haptisch möglich. Durch die abgerundete Form der Rückseite liegt die Vorrichtung außerdem besonders angenehm in der Hand.

Die vorliegende Erfindung soll unter Bezugnahme auf bevorzugte Ausführungsbeispiele näher erläutert werden. Dazu zeigen
- Figur 1: eine Vorrichtung zum Abdunsten von Wirkstoffen gemäß einem ersten Ausführungsbeispiel in geschlossener Stellung in einer Frontansicht;
- Figur 2: die Vorrichtung aus Figur 1 in einer Seitendarstellung;
- Figur 3: die Vorrichtung aus Figur 1 in geöffneter Stellung;
- Figur 4: die geöffnete Vorrichtung aus Figur 3 in einer Seitendarstellung;
- Figur 5: die Vorrichtung aus Figur 1 in einer seitlichen Schnittdarstellung in geschlossener Stellung;
- Figur 6: die Vorrichtung aus Figur 5 in geöffneter Stellung;
- Figur 7: die Vorrichtung aus Figur 5 in einer Frontansicht;
- Figur 8: die Vorrichtung aus Figur 7 in einer seitlichen Schnittdarstellung;
- Figur 9: die Vorrichtung aus Figur 5 in einer Seitendarstellung;
- Figur 10: die Vorrichtung aus Figur 5 in einer Schnittdarstellung;
- Figur 11: die Vorrichtung aus Figur 5 in einer weiteren Seitendarstellung;
- Figur 12: die Vorrichtung aus Figur 5 in einer weiteren Schnittdarstellung;
- Figur 13: die Vorrichtung aus Figur 5 in einer Seitendarstellung in geöffneter Stellung;
- Figur 14: die Vorrichtung aus Figur 5 in einer Schnittdarstellung in geöffneter Stellung;
- Figur 15: die Vorrichtung aus Figur 5 in einer weiteren Seitendarstellung;
- Figur 16: die Vorrichtung aus Figur 5 in einer weiteren Schnittdarstellung;
- Figur 17: die Vorrichtung aus Figur 5 in einer Frontansicht;
- Figur 18: die Vorrichtung aus Figur 5 in einer Querschnittdarstellung;
- Figur 19: eine Vorrichtung gemäß einem zweiten Ausführungsbeispiel in einer Schnittdarstellung;
- Figur 20: die Vorrichtung gemäß Figur 19 in geöffneter Stellung;
- Figur 21: die Vorrichtung gemäß Figur 19 in einer Rückansicht;
- Figur 22: die Vorrichtung gemäß Figur 19 in einer Querschnittdarstellung;
- Figur 23: die Vorrichtung gemäß Figur 19 in einer seitlichen Schnittdarstellung;
- Figur 24: die Vorrichtung gemäß Figur 19 in einer seitlichen Schnittdarstellung mit gedrücktem Befestigungselement;
- Figur 25: eine Vorrichtung gemäß einem dritten Ausführungsbeispiel in Draufsicht von einer ersten Seite;
- Figur 26: die Vorrichtung gemäß Figur 25 in Draufsicht von einer zweiten Seite;
- Figur 27: die Vorrichtung gemäß Figur 25 in einer Schnittdarstellung; und
- Figur 28: eine Variante der Vorrichtung gemäß Figur 25 in geöffneter Position in einer Schnittdarstellung.

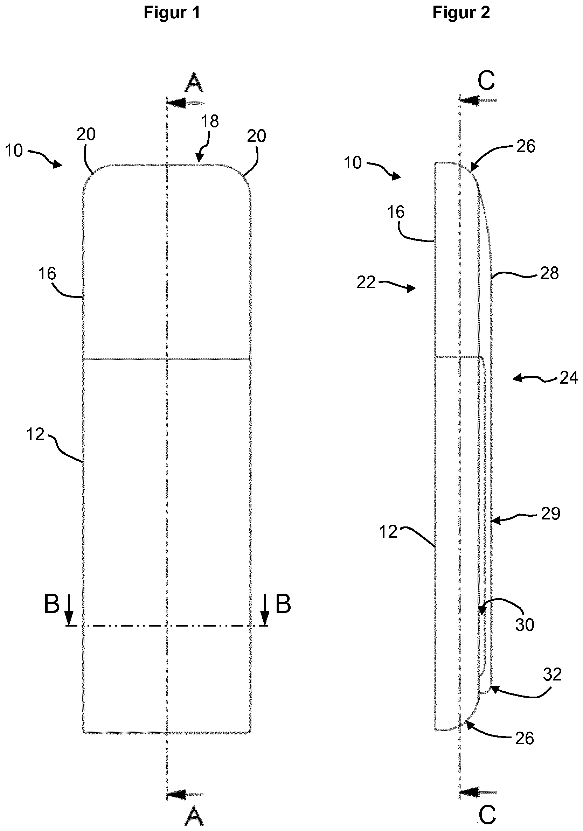

In den Figuren 1 und 2 ist eine Vorrichtung 10 zum Abdunsten von Wirkstoffen gemäß einem ersten Ausführungsbeispiel der vorliegenden Erfindung dargestellt. Figur 1 zeigt die Vorrichtung 10 in einer Frontdarstellung, Figur 2 in einer Seitendarstellung. Die Vorrichtung umfasst einen Behälter 12 für den Wirkstoff (nicht dargestellt) und ein Kappenelement 16, unter dem eine hier nicht sichtbare Verdunsteinheit angeordnet ist. Als Wirkstoff eignet sich beispielsweise ein ätherisches Öl, aber auch ein paramedizinischer Wirkstoff oder ein Parfum. Die Figuren 1 und 2 zeigen die Vorrichtung 10 im geschlossenen Zustand, mit dem Kappenelement 16 in geschlossener Position, in der die Verdunsteinheit 14 durch das Kappenelement 16 abgedeckt ist. Im geschlossenen Zustand kann die Vorrichtung 10 beispielsweise transportiert werden, ohne dass der Wirkstoff in dem Behälter 12 ungewollt entweicht.

Der Behälter 12 der Vorrichtung 10 ist länger als das Kappenelement 16. Dies kann dem Benutzer helfen zu erkennen, an welcher Seite sich der Behälter 12 und an welcher sich das Kappenelement 16 befindet. Außerdem weist das Kappenelement 16 an der dem Behälter 12 abgewandten Seite 18 abgerundete Ecken 20 auf. Diese Merkmale vereinfachen die Benutzung der Vorrichtung, da der Benutzer Behälter 12 und Kappenelement 16 leichter voneinander unterscheiden kann und direkt das Kappenelement 16 in eine geöffnete Position bewegen kann, in der er den Wirkstoff einatmen kann. In alternativen Ausführungsformen kann das Kappenelement 16 auch nicht abgerundete Ecken haben. Alternativ können der Behälter 12 und das Kappenelement 16 auch gleich lang ausgestaltet sein, oder der Behälter 12 kann kürzer als das Kappenelement 16 sein.

Bei dem hier gezeigten Ausführungsbeispiel verlaufen eine Vorderseite 22 und eine Rückseite 24 der Vorrichtung 10 im Wesentlichen parallel zueinander, wobei die Rückseite 24 in einem Übergangsbereich 26 zur Vorderseite eine abgerundete Form aufweist. Der Benutzer kann dadurch die Vorderseite 22 und die Rückseite 24 sowohl optisch als auch haptisch voneinander unterscheiden.

Außen an dem Kappenelement 16 ist ein Befestigungselement 28 in Form eines Clips angeordnet. Im hier dargestellten Ausführungsbeispiel verläuft ein Teil 29 des Befestigungselements 28 an der Rückseite 24 der Vorrichtung 10 teilweise mit einem Abstand 30 parallel zum Behälter 12. Das Befestigungselement 28 weist an einem Ende eine Verdickung 32 auf, die den Behälter 12 kontaktiert. Mit diesem Befestigungselement 28 kann die Vorrichtung 10 beispielsweise an Hemd- oder anderen Taschen befestigt werden. Die Verdickung 32 sorgt dabei für verbesserten Halt, da das Material, an dem die Vorrichtung 10 befestigt wird, zwischen der Verdickung 32 und dem Behälter 12 eingeklemmt wird. Die Vorrichtung 10 ist dadurch besser gesichert gegen ungewolltes Herausfallen aus der Tasche und ist zudem schnell griff- und einsatzbereit. Es können auch keine oder alternative Befestigungsmittel, wie beispielsweise eine Schlaufe, vorgesehen sein, die sich der Benutzer um den Hals hängen oder an Kleidung oder Taschen befestigen kann. Durch Druck auf den parallel zum Behälter 12 verlaufenden Teil 29 des Befestigungselements 28 und gleichzeitiges Schieben dieses Teils 29 parallel zum Behälter 12 kann das Kappenelement 16 verschoben werden. Die Vorrichtung 10 lässt sich dadurch besonders einfach einhändig bedienen.

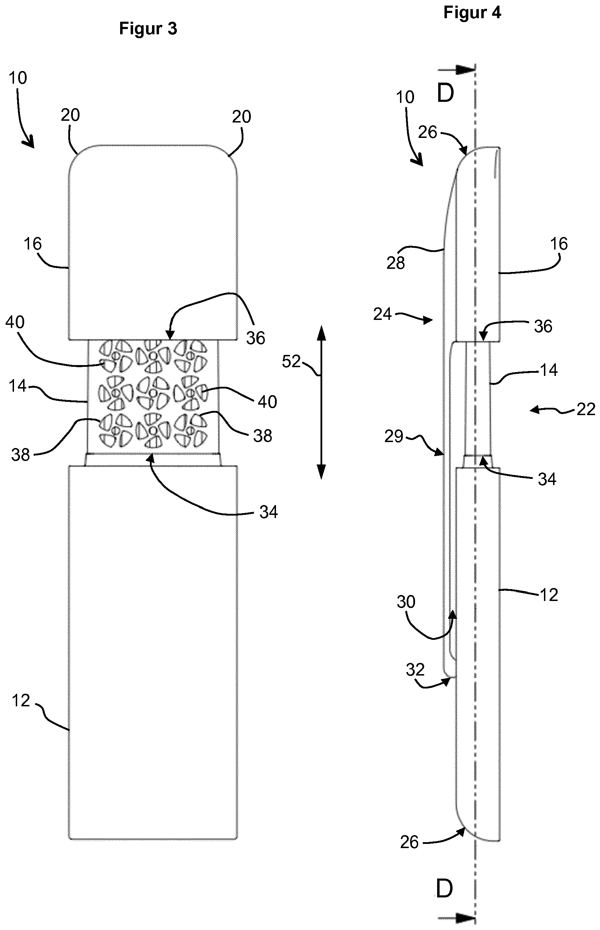

In Figur 3 und Figur 4 wird die Vorrichtung 10 von vorne und von der Seite mit dem Kappenelement 16 in einer vollständig geöffneten Position dargestellt. Die Verdunsteinheit 14 ist mit einer ersten Seite 34 an dem Behälter 12 angeordnet. An einer zweiten Seite 36 der Verdunsteinheit 14 ist das Kappenelement 16 angeordnet. Das Kappenelement 16 ist relativ zur Verdunsteinheit 14 in die in Figur 3 gezeigte geöffnete und in die in Figur 1 gezeigte geschlossene Position verschiebbar. Die Verschiebungsrichtung 52 des Kappenelements 16 ist dabei im Wesentlichen parallel zur Länge der Vorrichtung 10. In der geöffneten Position liegt die Verdunsteinheit 14 zum größten Teil frei und weist Öffnungen 38 auf, durch die der Wirkstoff abdunsten kann. In dieser Position kann der Benutzer die Vorrichtung 10 benutzen, um den Wirkstoff einzuatmen. Dazu hält er sich die Vorrichtung 10 mit dem Bereich der Öffnungen 38 der Verdunsteinheit 14 in beliebigem Abstand vor die Nase. Je geringer der Abstand zwischen der Verdunsteinheit 14 und der Nase des Benutzer ist, desto mehr Wirkstoff kann er einatmen. Die Öffnungen 38 können alternativ auch eine andere Form aufweisen, als hier dargestellt, beispielsweise rund oder eckig, und eine andere Größe oder verschiedene Formen in verschiedenen Größen haben. Hinter den Öffnungen 38 der Verdunsteinheit 14 sind im vorliegenden Beispiel Dochte 40 angeordnet, die den Wirkstoff transportieren. Anstelle der Dochte 40 ist unter anderem auch der Einsatz von einem oder mehreren Streifenelementen möglich. Die Verdunsteinheit 14 weist im hier dargestellten Ausführungsbeispiel Öffnungen 38 nur im Bereich der Vorderseite 22 der Vorrichtung 10 auf und ist im Bereich der Rückseite 24 geschlossen. In dieser Variante kann vermieden werden, dass der Wirkstoff im Bereich der Rückseite 24 herausläuft. In weiteren Varianten können auch zusätzlich im Bereich der Rückseite 24 Öffnungen angeordnet sein, durch die der Wirkstoff abgedunstet wird.

Durch die an der Rückseite 24 im Übergangsbereich 26 zur Vorderseite 22 abgerundete Form in Verbindung mit im Bereich der Vorderseite 22 angeordneten Öffnungen 38 an der Verdunsteinheit 14 kann der Benutzer leicht erkennen, wie er die Vorrichtung 10 halten und ausrichten muss, damit er den Wirkstoff nach dem Öffnen der Vorrichtung 10 einatmen kann. Wenn das Befestigungselement 28 an dem Kappenelement 16 so wie hier gezeigt auf der Rückseite 24 der Vorrichtung 10 angeordnet ist, weist die Verdunsteinheit 14 bevorzugt nur Öffnungen 38 im Bereich der Vorderseite 22 der Vorrichtung 10 auf, damit das Abdunsten und Aufnehmen des Wirkstoffs nicht durch das Befestigungselement 28 behindert wird.

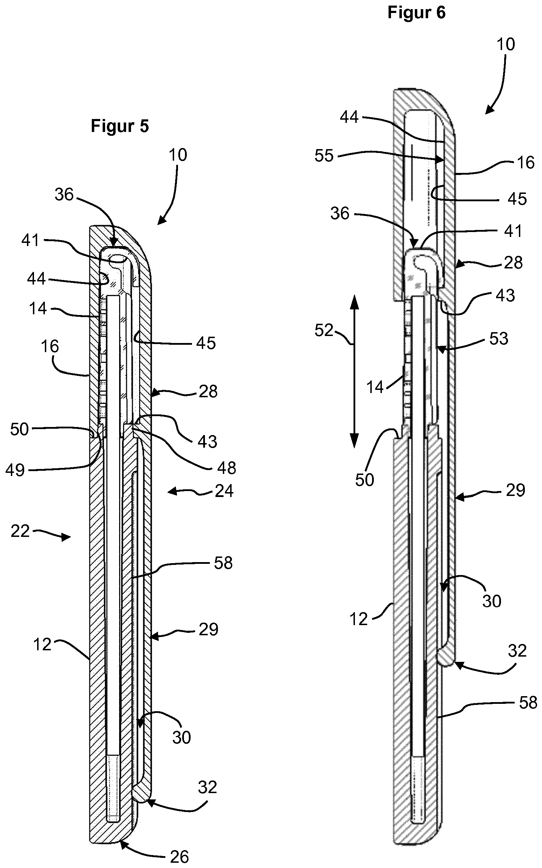

In den Figuren 5 und 6 wird die Vorrichtung 10 gemäß einem ersten Ausführungsbeispiel in einer seitlichen Schnittdarstellung entlang der Schnittlinie A-A aus Figur 1 in geschlossener und geöffneter Stellung dargestellt. In diesem Ausführungsbeispiel weist die Verschlussmechanik ein erstes Verschlussteil auf, das als Rasthaken 41 ausgebildet ist und an einer dem Behälter 12 für Wirkstoff abgewandten Seite 36 der Verdunsteinheit 14 angeordnet ist. Die Innenseite 44 des Kappenelements 16 weist in diesem Ausführungsbeispiel eine als Nut ausgebildete Vertiefung 45 als zweites Verschlussteil der Verschlussmechanik auf. Der Rasthaken 41 gleitet beim Bewegen zwischen geschlossener und geöffneter Stellung des Kappenelements 16 in der Vertiefung 45 und kontaktiert dabei die Innenseite 44 des Kappenelements 16. An der Innenseite 44 des Kappenelements 16 ist außerdem ein Anschlagelement 43 angeordnet, an dem der Rasthaken 41 in einer vollständig geöffneten Position des Kappenelements 16 anschlägt.

Bei der Vorrichtung 10 im hier dargestellten Ausführungsbeispiel weisen die Verdunsteinheit 14 und das Kappenelement 16 je eine zur Verschiebungsrichtung 52 des Kappenelements 16 im Wesentlichen parallele Seitenfläche 53, 55 auf. An der Seitenfläche 53 ist im Bereich der zweiten Seite 36 der Verdunsteinheit 14 der Rasthaken 41 angeordnet. An der Seitenfläche 55 des Kappenelements 16, an dessen Innenseite 44, sind die Vertiefung 45 und das Anschlagelement 43 für den Rasthaken 41 vorgesehen, in der dieser beim Bewegen des Kappenelements 16 gleiten kann bzw. an dem dieser bei vollständig geöffneter Position des Kappenelements 16 anschlägt. Die Seitenfläche 53 der Verdunsteinheit 14 kann in dem in den Figuren 5 und 6 gezeigten Ausführungsbeispiel als Rückseite der Verdunsteinheit 14 betrachtet werden. Der Rasthaken 41 gleitet in der Vertiefung 45, wenn das Kappenelement 16 geöffnet oder geschlossen wird, und wird dabei nicht zusammengedrückt. Das Anschlagelement 43 stellt das untere Ende der Vertiefung 45 dar, welches verhindert, dass das Kappenelement 16 von der Vorrichtung 10 entfernt werden kann. In Figur 6 befindet sich der Rasthaken 41 in einer Anschlagposition, in der das Kappenelement 16 vollständig geöffnet ist. Der Rasthaken 41 wird beim Zusammenbau auf Herstellerseite zusammengedrückt, nämlich um den Rasthaken 41 über das Anschlagelement 43 zu bewegen, wenn das Kappenelement 16 auf die Verdunsteinheit 14 aufgesteckt wird. Hat der Rasthaken 41 das Anschlagelement 43 passiert, entspannt er sich aufgrund der Vertiefung 45, in die er dabei eingreift. Dadurch kontaktiert er die Innenseite 44 des Kappenelements 16 derart, dass er beim Bewegen des Kappenelements 16 entlang der Innenseite 44 in der Vertiefung 45 gleiten kann und dass Kappenelement 16 dabei stabil geführt wird.

Bei dem hier dargestellten Ausführungsbeispiel sind das Kappenelement 16 und das Befestigungselement 28 einteilig ausgeformt. Die Vertiefung 45 ist teilweise an der zur Innenseite 44 des Kappenelements 16 weisenden Seite des Befestigungselements 28 angeordnet. Auf diese Weise wird der Raum, der sich durch die einteilige Ausführung am Übergang vom Kappenelement 16 zum Befestigungselement 28 ergibt, für die Verschlussmechanik genutzt, so dass die Vorrichtung 10 insgesamt eine besonders flache Bauweise aufweist.

In alternativen Ausführungsformen kann der Rasthaken 41 auch an der Innenseite 44 des Kappenelements 16 angeordnet sein und die entsprechende Vertiefung an der Seitenfläche 53 der Verdunsteinheit 14 vorgesehen sein, so dass der Rasthaken beim Bewegen zwischen geöffneter und geschlossener Position des Kappenelements 16 die Verdunsteinheit 14 parallel zur Verschiebungsrichtung 52 kontaktiert.

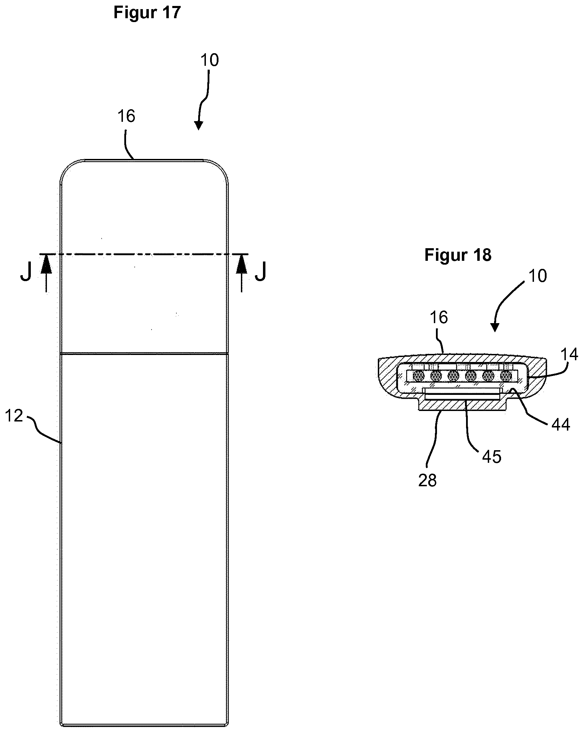

Außerdem weist die hier dargestellte Vorrichtung 10 eine Führungsnut 58 am Behälter 12 auf, die mit Bezug auf die Figuren 17 bis 20 näher erläutert wird.

Unterhalb der Vertiefung 45 kontaktiert das Kappenelement 16 den Behälter 12 an der Rückseite der Vorrichtung 10 im Bereich des Anschlagelements 43. Sowohl dort, als auch im übrigen Bereich, wie auch an der Vorderseite 22 der Vorrichtung 10 erkennbar, ist ein innerer Rand 48 des Behälters 12 höher ist als ein äußerer Rand 50, so dass der innere Rand 48 in der geschlossenen Position des Kappenelements 16 dessen Innenseite 44 kontaktiert. Der innere Rand 48 sowie die korrespondierende Kontaktfläche 49 an dem Kappenelement 16 sind außerdem schräg ausgeführt. Durch diese sogenannte Anlaufschräge wird das unerwünschte Abdampfen von Wirkstoff bei der geschlossenen Vorrichtung 10 minimiert. In alternativen Ausführungsformen kann zusätzlich oder alternativ eine O-Ring-Dichtung eingesetzt werden.

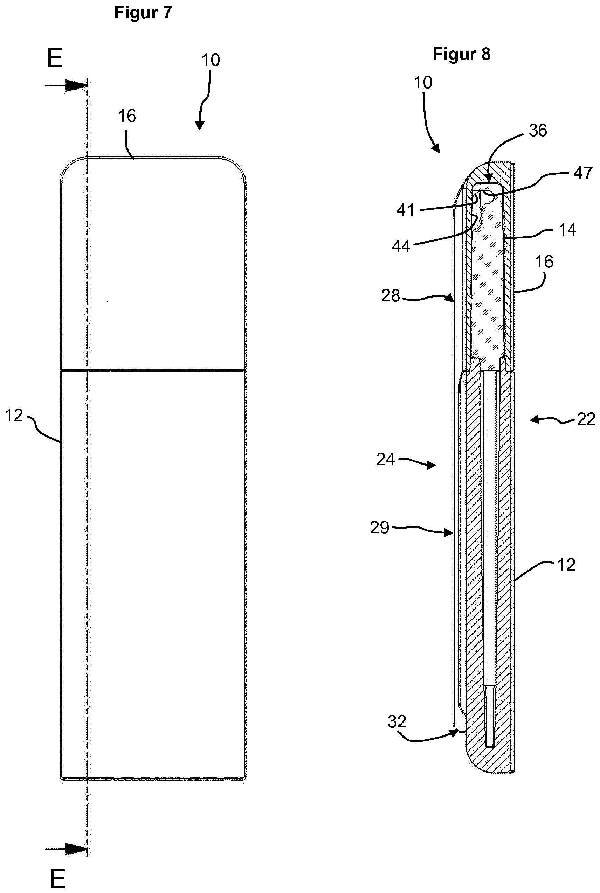

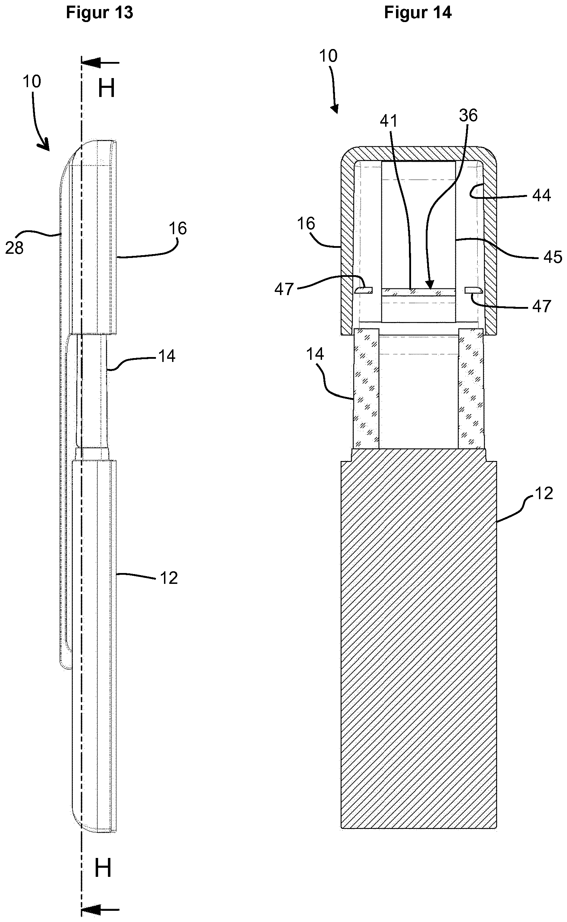

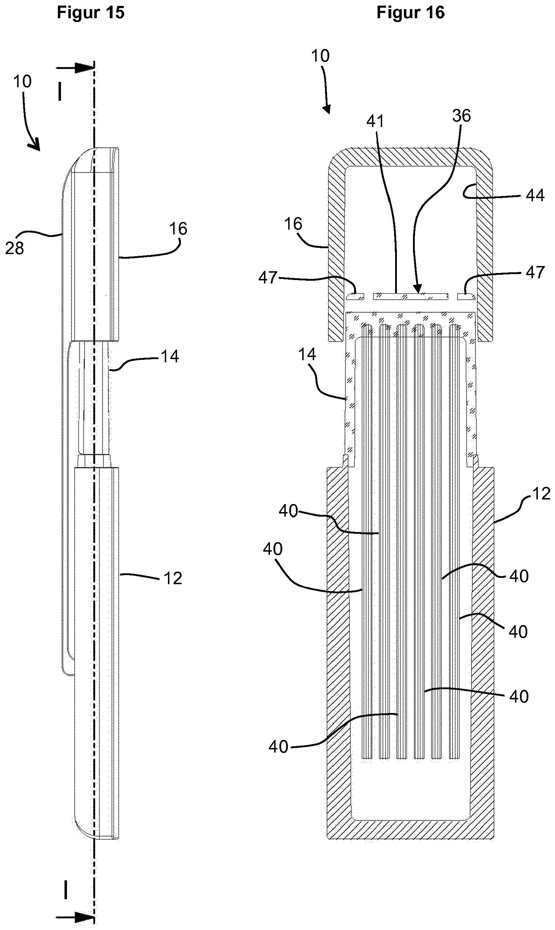

In Figur 7 und Figur 8 wird die Vorrichtung 10 von vorne und in einer Schnittdarstellung entlang der Schnittlinie E-E in der geschlossenen Stellung dargestellt. Die Schnittdarstellung entspricht im Wesentlichen der in Figur 5 gezeigten, mittigen Schnittdarstellung. Jedoch ist durch den außermittigen Schnitt der Vorrichtung 10 in Figur 8 im Bereich der zweiten Seite 36 der Verdunsteinheit 14 ein Führungselement 47 sichtbar, welches die Innenseite 44 des Kappenelements 16 kontaktiert. Das Führungselement 47 führt zu einer verbesserten Führung des Kappenelements 16, da auf diese Weise auch im Bereich neben dem Rasthaken 41, der in dieser Darstellung größtenteils durch Teile des Kappenelements 16 verdeckt wird, das heißt, dass im Wesentlichen über die gesamte Breite der Verdunsteinheit 14 ein gleichmäßiger Kontakt zum Kappenelement 16 aufrecht erhalten bleibt, wenn das Kappenelement 16 bewegt wird. Ein gleichartiges Führungselement 47 befindet sich auf der anderen Seite des Rasthakens 41, wie es beispielsweise in den im Folgenden beschriebenen Figuren 14 und 16 erkennbar ist.

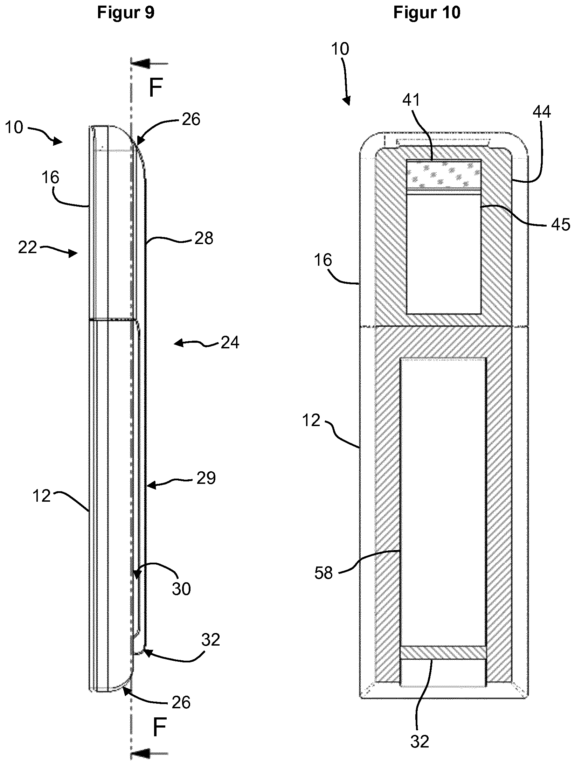

Die Figuren 9 und 10 zeigen die Vorrichtung 10 aus Figur 5 in einer Seitendarstellung, die im Wesentlichen Figur 2 entspricht, und in einer Schnittdarstellung entlang der Schnittlinie F-F. In der Schnittdarstellung ist ein Teil des Rasthakens 41 erkennbar, der sich in der Vertiefung 45 befindet. Da die Vorrichtung 10 im hier dargestellten Beispiel geschlossen ist, befindet sich der Rasthaken 41 im oberen Bereich der Vertiefung 45. Der Rasthaken 41 ist in diesem Beispiel etwas weniger als halb so breit wie das Kappenelement 16. Die Länge des Rasthakens 41 beträgt etwa ein Viertel der Länge der Vertiefung In alternativen Ausführungsformen kann der Rasthaken 41 auch schmaler oder breiter, sowie länger oder kürzer als hier dargestellt ausgeführt sein. Generell erlaubt eine größere Breite und Länge einen größeren Kontaktbereich in der Vertiefung 45 auf der Innenseite 44 des Kappenelements. Bevorzugt ist der Rasthaken 41 so lang ausgeführt, dass ein Hin- und Herwackeln senkrecht zur Verschiebungsrichtung 52 (Figur 3) des Kappenelements 16 minimiert wird und gleichzeitig die Verdunsteinheit 14 (nicht dargestellt) zur Benutzung freiliegt, wenn das Kappenelement 16 vollständig geöffnet ist.

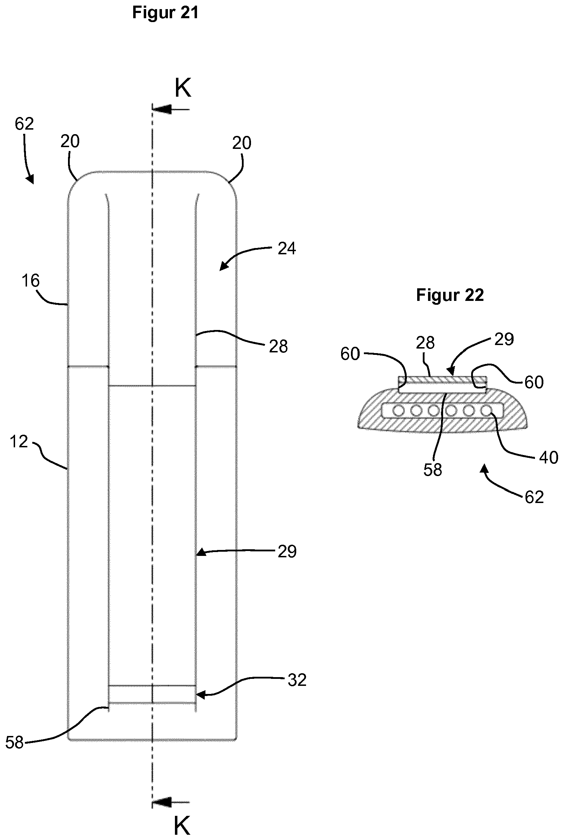

An dem Behälter 12 sind die Führungsnut 58 und die Verdickung 32 des Befestigungselements 28 erkennbar, in der das Befestigungselement 28 beim Öffnen und Schließen des Kappenelements 16 gleitet. Die Führungsnut 58 wird im Zusammenhang mit dem Befestigungselement 28 mit Bezug auf die Figuren 21 bis 24 näher beschrieben.

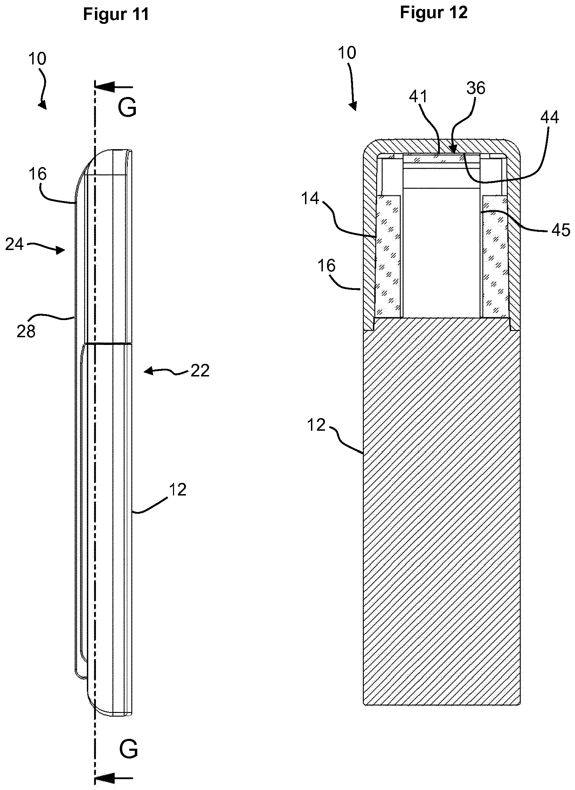

Die Figuren 11 und 12 zeigen die Vorrichtung 10 aus Figur 5 in einer weiteren Seitendarstellung bzw. in einer weiteren Schnittdarstellung entlang der Schnittlinie G-G. In dieser Darstellung ist ein weiterer Teil des Rasthakens 41 erkennbar, sowie die Vertiefung 45 und ein Teil der Verdunsteinheit 14. In der geschlossenen Stellung kontaktiert die Verdunsteinheit 14 in diesem Beispiel mit der zweiten Seite 36, vor allem im Bereich des Rasthakens 41, die Innenseite 44 des Kappenelements 16.

In Figur 13 ist eine weitere Seitenansicht der Vorrichtung 10 in der geöffneten Position gezeigt, und in Figur 14 ist die Vorrichtung 10 ebenfalls in geöffneter Stellung in einer Schnittansicht entlang der Schnittlinie H-H dargestellt. In der Schnittdarstellung ist ein Teil des Rasthakens 41 sichtbar, der sich in der Vertiefung 45 in der Anschlagposition befindet, da das Kappenelement 16 vollständig geöffnet ist. Ein Teil der Verdunsteinheit 14, der sich noch innerhalb des Kappenelements 16 befindet, kontaktiert dabei die Innenseite 44 des Kappenelements 16, wodurch das Kappenelement 16 zusätzlich entlang der Verdunsteinheit 14 geführt wird. In diesem Ausführungsbeispiel sind an der Verdunsteinheit 14 neben dem Rasthaken 41 an beiden Seiten Führungselemente 47 angeordnet, die die Innenseite 44 des Kappenelements 16 kontaktieren und dadurch für eine besonders stabile Führung des Kappenelements 16 beim Öffnen und Schließen der Vorrichtung 10 sorgen.

In alternativen Ausführungsformen der Vorrichtung 10 können an der Innenseite 44 des Kappenelements 16 zusätzliche oder alternative Führungselemente für den Rasthaken 41 angeordnet sein.

Figur 15 zeigt die Vorrichtung 10 in einer Seitenansicht in der geöffneten Position, die im Wesentlichen Figur 13 entspricht, und Figur 16 zeigt die Vorrichtung 10 entlang der Schnittlinie I-I. In Figur 16 sind neben dem Rasthaken 41 und den Führungselementen 47 im Bereich der zweiten Seite 36 der Verdunsteinheit 14 auch sechs Dochte 40 dargestellt, die vom Behälter 12 in die Verdunsteinheit 14 reichen. Die Führungselemente 47 kontaktieren die Innenseite 44 des Kappenelements 16, so dass das Kappenelement 16 beim Bewegen zwischen der geschlossenen und geöffneten Position besonders stabil geführt wird.

In den Figuren 17 und 18 ist die Vorrichtung 10 gemäß dem ersten Ausführungsbeispiel in einer Frontansicht, die im Wesentlichen der Figur 1 entspricht, und einer Querschnittdarstellung entlang der Schnittlinie J-J dargestellt. In der Schnittdarstellung ist erkennbar, dass das Kappenelement 16 und das Befestigungselement 28 einteilig ausgeformt sind. Die Vertiefung 45 ist an der zur Innenseite 44 des Kappenelements 16 weisenden Seite des Befestigungselements 28 angeordnet. Dadurch ist ausreichend Platz für die Verschlussmechanik und die Vorrichtung 10 weist nur eine geringe Dicke auf.

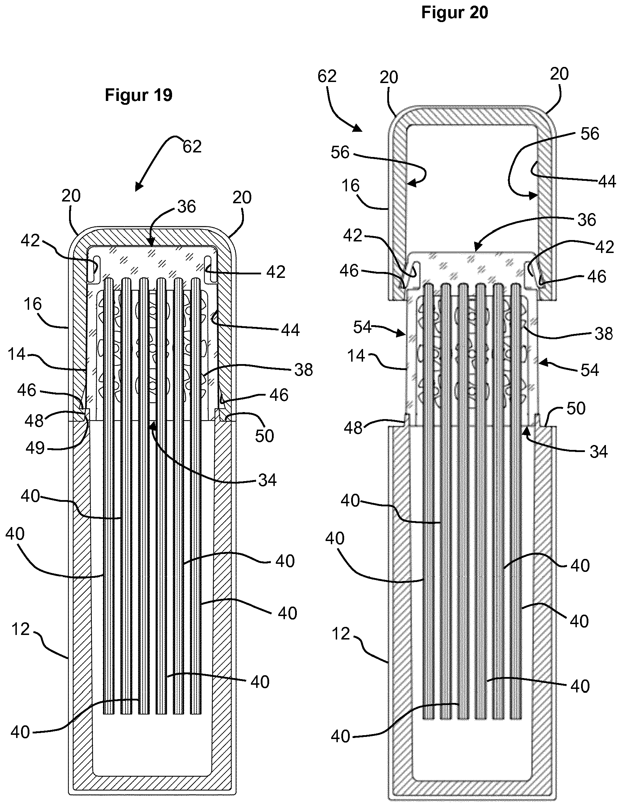

In Figur 19 ist eine Schnittdarstellung einer Vorrichtung 62 gemäß einem zweiten Ausführungsbeispiel der vorliegenden Erfindung. In der äußeren Ansicht entspricht die Vorrichtung 62 gemäß dem zweiten Ausführungsbeispiel der Vorrichtung 10 gemäß dem ersten Ausführungsbeispiel, so dass die Schnittlinie C-C aus Figur 2 auf das zweite Ausführungsbeispiel übertragen werden kann. Das Kappenelement 16 befindet sich in der geschlossenen Position, so dass die Verdunsteinheit 14 abgedeckt ist. Die mit der ersten Seite 34 an dem Behälter 12 angeordnete Verdunsteinheit 14 weist an der zweiten Seite 36 zwei erste Verschlussteile auf, die als Rastnasen 42 ausgeformt sind. Die Rastnasen 42 kontaktieren das Kappenelement 16 an dessen Innenseite 44 und werden in dieser Stellung zusammengedrückt. An der Innenseite 44 des Kappenelements 16 sind außerdem zwei als Vertiefungen 46 ausgeformte zweite Verschlussteile für die Rastnasen 42 angeordnet. Die Rastenasen 42 und Vertiefungen 46 ergeben eine Verschlussmechanik mit zueinander komplementären Verschlussteilen, durch die die Haptik und Führung des Kappenelements 16 beim Öffnen und Schließen der Vorrichtung 62 verbessert ist.

In alternativen Ausführungsformen können eine oder zwei Rastnasen auch an der Innenseite 44 des Kappenelements 16 angeordnet sein. Eine oder mehrere entsprechende, komplementäre Vertiefungen können dann an der Verdunsteinheit 14 angeordnet sein, so dass die Rastnasen des Kappenelements 16 die Verdunsteinheit 14 beim Bewegen des Kappenelements 16 kontaktieren und in der vollständig geöffneten Position in die Vertiefungen an der Verdunsteinheit einrasten. Alternativ können auch ein oder mehrere andere geeignete erste Verschlussteile an der Verdunsteinheit und zweite Verschlussteile an der Innenseite des Kappenelements vorgesehen sein, die zueinander komplementär sind und derart zusammenwirken, dass sie bei einer vollständig geöffneten Position des Kappenelements an dessen Innenseite ineinander verrasten und beim Bewegen zwischen geöffneter und geschlossener Position und in der geschlossenen Position des Kappenelements das oder jedes erste Verschlussteil die Innenseite des Kappenelements oder das oder jedes zweite Verschlussteil die Verdunsteinheit parallel zur Verschiebungsrichtung des Kappenelements kontaktiert, vorgesehen sein.

Unterhalb der Vertiefungen 46 kontaktiert das Kappenelement 16 den Behälter 12, wobei ein innerer Rand 48 des Behälters 12 höher ist als ein äußerer Rand 50, so dass der innere Rand 48 in der geschlossenen Position des Kappenelements 16 dessen Innenseite 44 kontaktiert. Der innere Rand 48 sowie die korrespondierende Kontaktfläche 49 an dem Kappenelement 16 sind außerdem schräg ausgeführt. Durch diese sogenannte Anlaufschräge wird das unerwünschte Abdampfen von Wirkstoff, d. h. der Verlust von Wirkstoff, bei der geschlossenen Vorrichtung 62 minimiert. In alternativen Ausführungsformen kann zusätzlich oder alternativ eine O-Ring-Dichtung eingesetzt werden.

Im hier dargestellten Beispiel reichen sechs Dochte 40 von dem Behälter 12, in dem sich der Wirkstoff (nicht dargestellt) befindet, in die Verdunsteinheit 14. Sie transportieren den Wirkstoff, so dass dieser über die Öffnungen 38 der Verdunsteinheit 14 abgedunstet werden kann. Anstelle von sechs Dochten 40 können alternativ auch weniger oder mehr Dochte eingesetzt werden, beispielsweise ein, zwei, drei, vier, fünf, sieben, acht oder mehr Dochte. Es können auch z. B. ein oder mehrere Dochte vorgesehen sein, die nicht nur mit einem, sondern mit beiden Enden in die Verdunsteinheit 14 reichen und im Behälter 12 einen oder je nach Länge des Dochts gegebenenfalls mehrere Bögen aufweisen.

Figur 20 zeigt die Vorrichtung 62 mit dem Kappenelement 16 in geöffneter Position in einer Schnittdarstellung entlang der Schnittlinie D-D aus Figur 4. Die Schnittlinie D-D kann auf das zweite Ausführungsbeispiel übertragen werden, da sich die Vorrichtungen 10, 62 äußerlich nicht unterscheiden. Das Kappenelement 16 befindet sich in der vollständig geöffneten Position, die Rastnasen 42 sind in die Vertiefungen 46 an der Innenseite 44 des Kappenelements 16 eingerastet. Das Kappenelement 16 kann somit nicht über diese Vertiefungen 46 hinaus geöffnet oder von der Verdunsteinheit 14 entfernt werden. Dadurch kann auch verhindert werden, dass das Kappenelement 16 verloren geht.

Bei der Vorrichtung 62 im hier dargestellten Beispiel weisen die Verdunsteinheit 14 und das Kappenelement 16 je zwei zur Verschiebungsrichtung 52 (Figur 3) des Kappenelements 16 im Wesentlichen parallele Seitenflächen 54, 56 auf. An den beiden Seitenflächen 54 ist im Bereich der zweiten Seite 36 der Verdunsteinheit 14 je eine Rastnase 42 angeordnet. An den beiden Seitenflächen 56 des Kappenelements 16, an dessen Innenseite 44, ist je eine Vertiefung 46 für eine der Rastnasen 42 vorgesehen. In alternativen Ausführungsformen kann auch nur an einer der Seitenflächen 54, 56 oder an weiteren entsprechenden, zur Bewegungsrichtung 52 parallelen Seitenflächen eine Rastnase 42 bzw. eine Vertiefung 46 so angeordnet sein, dass die Rastnase 42 in der vollständig geöffneten Position des Kappenelements 16 in der Vertiefung 46 einrastet, und dass die Rastnase 42 beim Bewegen des Kappenelements 16 zwischen geöffneter und geschlossener Position und in der geschlossenen Position des Kappenelements 16 die Innenseite 44 des Kappenelements 16 parallel zur Verschiebungsrichtung 52 kontaktiert.

Die zwei Seitenflächen 54, 56 der Verdunsteinheit 14 und des Kappenelements 16, an denen die Rastnasen 42 bzw. die Vertiefung 46 angeordnet sind, ergeben sich aufgrund der im Wesentlichen quaderförmigen Ausgestaltung der Vorrichtung 10, bei der die Tiefe der Vorrichtung 62 kleiner ist als die Länge und Breite. Die Rastnasen 42 werden beim Bewegen des Kappenelements 16 zwischen geöffneter und geschlossener Position gegen die Seitenflächen 56 an der Innenseite 44 des Kappenelements 16 gedrückt und werden so zwischen der Innenseite 44 an Vorder- und Rückseite 22, 24 der Vorrichtung 62 und den Seitenflächen 56 parallel zur Verschiebungsrichtung 52 geführt. Dies ergibt eine stabile Führung des Kappenelements 16, ein Hin- und Herwackeln des Kappenelements 16 beim Verschieben wird durch den gleichmäßigen Druck der Rastnasen 42 weitgehend verhindert.

In alternativen Ausführungsformen kann die Vorrichtung auch zylindrisch ausgestaltet sein, wobei der Durchmesser im Vergleich zur Länge deutlich kleiner sein kann. Bei einer derartigen Form ist die innere Mantelfläche des Kappenelements und die äußere Mantelfläche der Verdunsteinheit als Seitenflächen aufzufassen, an denen mindestens eine Vertiefung bzw. mindestens eine Rastnase oder alternativ andere zueinander komplementäre erste und zweite Verschlussteile angeordnet sind. Um beim Öffnen der Vorrichtung das ineinander Verrasten der Verschlussteile zu gewährleisten, können entsprechende Führungselemente zum Führen des Kappenelements entlang der Verdunsteinheit an der Innenseite des Kappenelements bzw. der Außenseite der Verdunsteinheit vorgesehen sein
Figur 21 zeigt die Vorrichtung 62 gemäß dem zweiten Ausführungsbeispiel in einer Rückansicht. Auf der Rückseite 24 der Vorrichtung 62 ist außen an dem Kappenelement 16 das Befestigungselement 28 angeordnet, welches teilweise entlang des Behälters 12 verläuft. Unterhalb der Verdickung 32 ist noch eine Führungsnut 58 am Behälter 12 erkennbar. Deutlicher ist die Führungsnut 58 in Figur 22 dargestellt, die einer Querschnittdarstellung entlang der Schnittlinie B-B aus Figur 1 entspricht. Die Führungsnut 58 ist bevorzugt so breit, dass zumindest die Verdickung 32 des Befestigungselements 28 beim Verschieben des Kappenelements 16 in der Führungsnut 58 gleiten kann. Vorteilhafterweise ermöglichen die seitlichen Randflächen 60 der Führungsnut 58 eine seitliche Führung des Befestigungselements 28. Durch Druck auf den parallel zum Behälter 12 verlaufenden Teil 29 des Befestigungselements 28, wobei der Druck eine Kraftkomponente in Richtung des Behälters 12 und eine parallel zum Behälter 12 aufweist, kann das Kappenelement 16 verschoben werden. Auf diese Weise kann die Vorrichtung 62 auch einhändig geöffnet und geschlossen werden. Der parallel zum Behälter 12 verlaufende Teil 29 des Befestigungselements 28 kann zumindest teilweise drückbar ausgestaltet sein, wie im folgenden mit Bezug auf Figur 20 beschrieben wird. In dem Behälter 12 sind außerdem die sechse Dochte 40 im Querschnitt dargestellt.

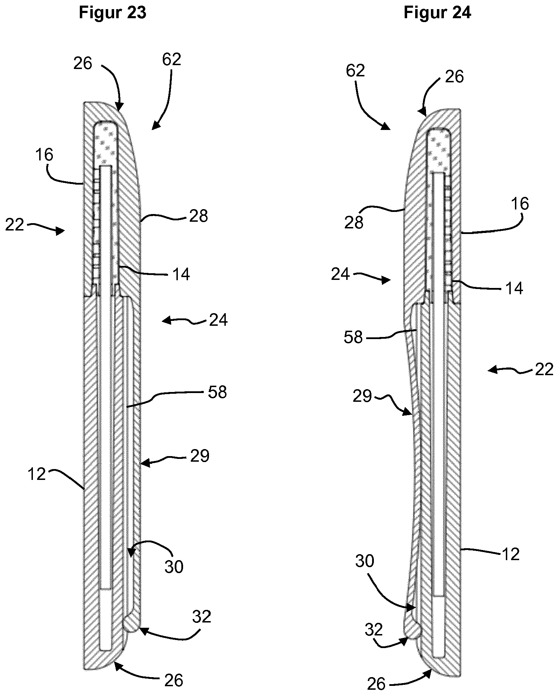

In den Figuren 23 und 24 wird die Vorrichtung 62 gemäß dem zweiten Ausführungsbeispiel im Schnitt entlang der Schnittlinie A-A aus Figur 1 bzw. der Schnittlinie K-K aus Figur 18 dargestellt. Die Schnittlinie A-A ist auf das zweite Ausführungsbeispiel übertragbar, da sich die Vorrichtungen 10, 62 äußerlich nicht unterscheiden. Das Kappenelement 16 der Vorrichtung 62 ist in der geschlossenen Position. In Figur 23 ist der parallel zum Behälter 12 verlaufende Teil 29 des Befestigungselements 28 nicht eingedrückt, so dass das Befestigungselement 28 den Behälter 12 nur mit der Verdickung 32 kontaktiert, die sich in der Führungsnut 58 befindet. In Figur 24 ist der parallel zum Behälter 12 verlaufende Teil 29 des Befestigungselements 28 teilweise eingedrückt dargestellt. Das Eindrücken erfolgt beispielsweise durch einen Finger des Benutzers zum Bewegen des Kappenelements 16 in die geöffnete Position. Auch zum Schließen der Vorrichtung 62 aus der geöffneten Position des Kappenelements 16 kann der Teil 29 des Befestigungselements 28 eingedrückt werden. Durch das Drücken des parallel zum Behälter 12 verlaufenden Teils 29 des Befestigungselements 28 wird dieses gegen den Behälter 12, und im hier gezeigten Ausführungsbeispiel in die Führungsnut 58, gedrückt. Dabei kann ein Teil des Befestigungselements 28 den Behälter 12 kontaktierten. Wenn der Benutzer gleichzeitig einen Druck in die Verschiebungsrichtung 52 (Figur 3) parallel zum Behälter 12 auf das Befestigungselement 28 ausübt, wird das Kappenelement 16 zwischen der geöffneten und geschlossenen Position verschoben. Der parallel zum Behälter 12 verlaufende Teil 29 des Befestigungselements 28 kann dann in der Führungsnut 58 gleiten und das einhändige Öffnen und Schließen der Vorrichtung 62 wird erleichtert. Der drückbare, im Sinne von gegen den Behälter 12 drückbar, parallel zum Behälter 12 verlaufende Teil 29 des Befestigungselements 28 ist bevorzugt aus elastischen Materialien, beispielsweise Kunststoffen, gefertigt. Diese ermöglichen, dass das Befestigungselement 28 einerseits einfach gegen den Behälter 12 gedrückt werden kann, sich aber auch schnell wieder in die Ursprungsstellung zurückbewegen kann, in der das Befestigungselement 28 wieder beabstandet zum Behälter 12 positioniert ist, wie z. B. in Figur 23 gezeigt, sobald der Benutzer keinen Druck mehr auf das Befestigungselement 28 ausübt. Das Material des parallel zum Behälter 12 verlaufenden Teils 29 des Befestigungselements 28 kann sich auch von dem Material des restlichen Befestigungselements 28 unterscheiden.

Im hier dargestellten zweiten Ausführungsbeispiel der Vorrichtung 62 sind das Kappenelement 16 und das Befestigungselement 28 einteilig ausgeformt. In alternativen Ausführungsformen kann das Befestigungselement 28 auch separat an das Kappenelement 16 angeformt sein. Dabei kann es aus dem gleichen oder auch aus einem anderen Material als das Kappenelement 16 gefertigt sein.

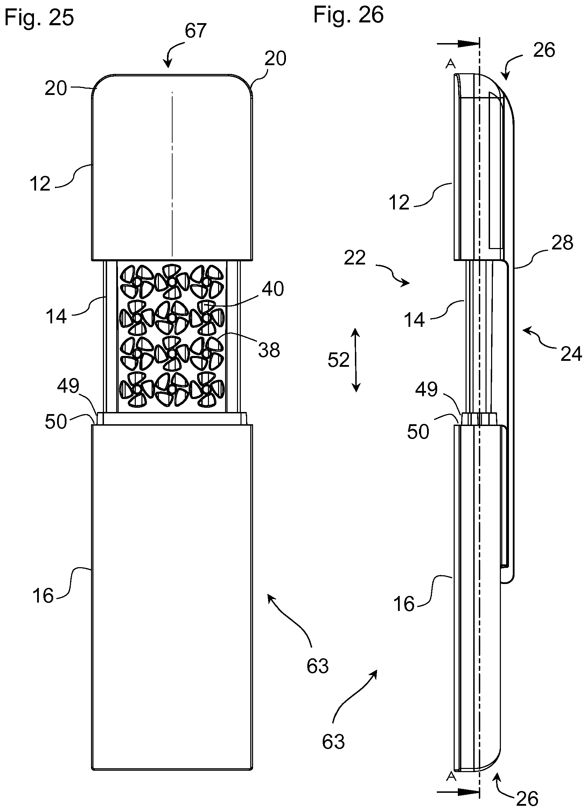

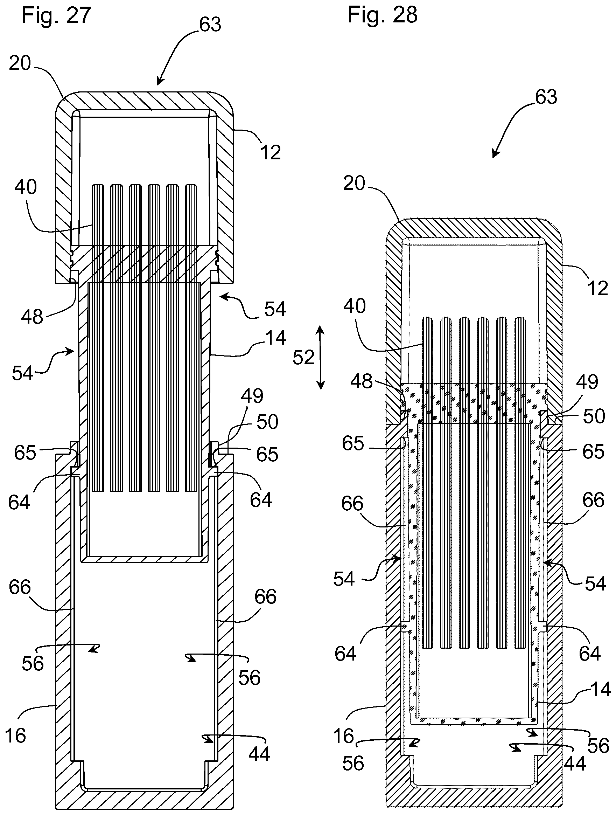

In den Figuren 25 bis 28 ist eine Vorrichtung 63 gemäß einem dritten Ausführungsbeispiel schematisch dargestellt. In den Figuren 25 und 26 ist die Vorrichtung 63 von vorne und von der Seite jeweils in vollständig geöffneter Position dargestellt. In Figur 27 ist ein Schnitt längs der Linie A-A aus Figur 26 dargestellt. In Figur 28 ist ein entsprechender Schnitt bei einer Vorrichtung 63 in geschlossener Position dargestellt.

Im hier dargestellten Beispiel ist der Wirkstoffbehälter 12 kleiner als das Kappenelement 16. Das bereits in Verbindung mit den vorherigen Ausführungsformen erläuterte Befestigungselement 28 ist in diesem Ausführungsbeispiel statt am Kappenelement 16 am Behälter 12 angeordnet und der Behälter 12 weist am der dem Kappenelement 16 abgewandten Seite 67 abgerundete Ecken 20 auf, um Behälter 12 und Kappenelement 16 für den Benutzer unterscheidbarer zu machen. Bei dem hier gezeigten Ausführungsbeispiel verlaufen eine Vorderseite 22 und eine Rückseite 24 der Vorrichtung 10 im Wesentlichen parallel zueinander, wobei die Rückseite 24 in einem Übergangsbereich 26 zur Vorderseite eine abgerundete Form aufweist. Der Benutzer kann dadurch die Vorderseite 22 und die Rückseite 24 sowohl optisch als auch haptisch voneinander unterscheiden. Die Verdunsteinheit 14 mit Öffnungen 38 und Dochten 40 ist im hier dargestellten Beispiel mit dem Behälter 12 starr verbunden und kann durch Hin- und Herschieben des Kappenelements 16 in Bewegungsrichtung 52 vom Kappenelement 16 bedeckt oder offengelegt werden.

Das Kappenelement 16 weist einen umlaufenden Rand 50 mit einer Kontaktfläche 59 auf, der in geschlossener Position mit einen komplementären umlaufenden inneren Rand (siehe 48 in Figur 27) des Behälters 12 in Kontakt tritt. Der innere Rand 48 sowie die korrespondierende Kontaktfläche 49 an dem Kappenelement 16 sind leicht schräg ausgeführt. Durch diese sogenannte Anlaufschräge wird das unerwünschte Abdampfen von Wirkstoff bei der geschlossenen Vorrichtung 10 minimiert. In alternativen Ausführungsformen kann zusätzlich oder alternativ eine O-Ring-Dichtung eingesetzt werden.

Im hier dargestellten Beispiel weisen die Verdunsteinheit 14 und das Kappenelement 16 je zwei zur Verschiebungsrichtung 52 des Kappenelements 16 im Wesentlichen parallele Seitenflächen 54, 56 auf. An den beiden Seitenfläche 54 der Verdunsteinheit 14 ist beabstandet zum Behälter 12 je ein Vorsprung angeordnet. An den beiden innenliegenden Seitenflächen 56 des Kappenelements 16 ist je eine Führungsrille 66 für eine der Vorsprünge 64 vorgesehen. Die Vorsprünge 64 gleiten beim Bewegen zwischen geschlossener und geöffneter Stellung des Kappenelements 16 in den Führungsrillen 66 und kontaktieren dabei die Innenseite 44 des Kappenelements 16. An der Innenseite 44 des Kappenelements 16 ist außerdem für jedes Vorsprung-Führungsrillen-Paar ein Anschlagelement 65 angeordnet, an dem der jeweilige Vorsprung 64 in einer vollständig geöffneten Position des Kappenelements 16 anschlägt. Beim Bewegen des Kappenelements 16 zwischen geöffneter und geschlossener Position bleiben die Vorsprünge 64 der Verdunsteinheit 14 in den Führungsrillen 66 mit dem Kappenelement in Kontakt. Dies ergibt eine stabile Führung des Kappenelements 16, ein Hin- und Herwackeln des Kappenelements 16 beim Verschieben wird durch den gleichmäßigen Druck der Rastnasen 42 weitgehend verhindert. Die Kraft, die vom Benutzer aufgewendet werden muss, um die Vorrichtung 63 zu öffnen oder zu schließen, kann beispielsweise über die Toleranz in den Abmessungen der Führungsrillen 66 und Vorsprünge 64 sowie über die Materialwahl beeinflusst werden. Diese und andere Parameter wirken sich auf die Haft- und Gleitreibung zwischen Verdunsteinheit 14 und Kappenelement 16 aus. Ferner können ein oder mehrere leichte Verengungen in der oder den Führungsrillen 66, die eine oder mehrere unterschiedliche Positionen von Kappenelement 16 zu Verdunsteinheit 14 definieren. Um diese Positionen zu verlassen, muss der Benutzer etwas mehr Kraft zur Bewegung des Kappenelements 16 aufwenden. Vorzugsweise ist diese Kraft größer als die auf das Kappenelement 16 oder-je nach Orientierung im Raum - den Behälter 12 mit Verdunsteinheit 14 wirkende Schwerkraft. So unterscheiden sich die Vorrichtungen 63 aus Figur 27 und Figur 28 dahingehend, das die Verdunsteinheit 14 aus jeweils unterschiedlichem Material gefertigt ist.

In alternativen Ausführungsformen kann auch nur an einer der Seitenflächen 54, 56 oder an weiteren entsprechenden, zur Bewegungsrichtung 52 parallelen Seitenflächen ein Vorsprung 64 bzw. eine Führungsrille 66 angeordnet sein. Die zwei Seitenflächen 54, 56 der Verdunsteinheit 14 und des Kappenelements 16, an denen die Vorsprünge 64 bzw. die Führungsrillen 66 angeordnet sind, ergeben sich aufgrund der im Wesentlichen quaderförmigen Ausgestaltung der Vorrichtung 63, bei der die Tiefe der Vorrichtung 63 kleiner ist als die Länge und Breite. In alternativen Ausführungsformen kann die Vorrichtung auch zylindrisch ausgestaltet sein, wobei der Durchmesser im Vergleich zur Länge deutlich kleiner sein kann. Bei einer derartigen Form ist die innere Mantelfläche des Kappenelements und die äußere Mantelfläche der Verdunsteinheit als Seitenflächen aufzufassen, an denen mindestens eine Führungsrille bzw. mindestens Vorsprung oder alternativ andere zueinander komplementäre erste und zweite Verschlussteile angeordnet sind.

### Bezugszeichenliste

- 10: Vorrichtung
- 12: Behälter
- 14: Verdunsteinheit
- 16: Kappenelement
- 18: abgewandte Seite
- 20: abgerundete Ecke
- 22: Vorderseite
- 24: Rückseite
- 26: Übergangsbereich
- 28: Befestigungselement
- 29: parallel verlaufender Teil
- 30: Abstand
- 32: Verdickung
- 34, 36: erste bzw. zweite Seite
- 38: Öffnungen
- 40: Dochte
- 41: Rasthaken
- 42: Rastnase(n)
- 43: Anschlagelement
- 44: Innenseite
- 45: Vertiefung
- 46: Vertiefung(en)
- 47: Führungselement(e)
- 48: innerer Rand
- 49: Kontaktfläche
- 50: äußerer Rand
- 52: Verschiebungsrichtung
- 53-56: Seitenfläche
- 58: Führungsnut
- 60: Randflächen
- 62, 63: Vorrichtung
- 64: Vorsprung
- 65: Anschlagelement
- 66: Führungsrille
- 67: Seite

## Patentansprüche

1. Vorrichtung zum Abdunsten von Wirkstoffen, umfassend einen Behälter(12) für den Wirkstoff und eine Verdunsteinheit (14), an der ein Kappenelement (16) angeordnet ist, das relativ zur Verdunsteinheit in eine geöffnete und in eine geschlossene Position verschiebbar ist, wobei die Verdunsteinheit in der geschlossenen Position durch das Kappenelement abgedeckt ist und in der geöffneten Position zumindest teilweise freiliegt, wobei die Vorrichtung eine Verschlussmechanik aufweist, umfassend ein erstes Verschlussteil, das an der Verdunsteinheit (14) angeordnet ist, und ein zweites, zum ersten Verschlussteil komplementäres Verschlussteil, das an einer Innenseite (44) des Kappenelements (16) angeordnet ist, wobei die Verschlussteile derart zusammenwirken, dass sich das erste oder das zweite Verschlussteil bei einer vollständig geöffneten Position des Kappenelements (16) in einer Anschlagposition befindet und beim Bewegen zwischen geöffneter und geschlossener Position des Kappenelements (16) das erste Verschlussteil die Innenseite (44) des Kappenelements (16) oder das zweite Verschlussteil die Verdunsteinheit (14) parallel zur Verschiebungsrichtung (52) kontaktiert, **dadurch gekennzeichnet, dass** das erste Verschlussteil als Rasthaken (41) ausgebildet ist, der an einer dem Behälter (12) für Wirkstoff abgewandten Seite (36) der Verdunsteinheit (14) angeordnet ist, dass das Kappenelement (16) an der Innenseite (44) eine als Nut ausgebildete Vertiefung (45) aufweist, in der der Rasthaken (41) beim Bewegen zwischen geöffneter und geschlossener Position des Kappenelements (16) gleitet, und dass an der Innenseite (44) des Kappenelements (16) ein Anschlagelement (43) für den Rasthaken (41) angeordnet ist, an dem der Rasthaken (41) in einer vollständig geöffneten Position des Kappenelements (16) anschlägt.

2. Vorrichtung nach Anspruch 1, **dadurch gekennzeichnet, dass** die Verdunsteinheit (14) und das Kappenelement (16) je eine zur Verschiebungsrichtung (52) im Wesentlichen parallele Seitenfläche aufweisen, dass der Rasthaken (41) an der Seitenfläche der Verdunsteinheit (14) angeordnet ist, und dass die Vertiefung (45) und das Anschlagelement (43) an der Seitenfläche des Kappenelements (16) angeordnet sind.

3. Vorrichtung nach Anspruch 1 oder 2, **dadurch gekennzeichnet, dass** an der Verdunsteinheit (14) an mindestens einer der Innenseite (44) des Kappenelements (16) zugewandten Seite ein Führungselement (47) angeordnet ist.

4. Vorrichtung zum Abdunsten von Wirkstoffen, umfassend einen Behälter (12) für den Wirkstoff und eine Verdunsteinheit (14), an der ein Kappenelement (16) angeordnet ist, das relativ zur Verdunsteinheit in eine geöffnete und in eine geschlossene Position verschiebbar ist, wobei die Verdunsteinheit in der geschlossenen Position durch das Kappenelement abgedeckt ist und in der geöffneten Position zumindest teilweise freiliegt, wobei die Vorrichtung eine Verschlussmechanik aufweist, umfassend ein erstes Verschlussteil, das an der Verdunsteinheit (14) angeordnet ist, und ein zweites, zum ersten Verschlussteil komplementäres Verschlussteil, das an einer Innenseite (44) des Kappenelements (16) angeordnet ist, wobei die Verschlussteile derart zusammenwirken, dass sich das erste oder das zweite Verschlussteil bei einer vollständig geöffneten Position des Kappenelements (16) in einer Anschlagposition befindet und beim Bewegen zwischen geöffneter und geschlossener Position des Kappenelements (16) das erste Verschlussteil die Innenseite (44) des Kappenelements (16) oder das zweite Verschlussteil die Verdunsteinheit (14) parallel zur Verschiebungsrichtung (52) kontaktiert, **dadurch gekennzeichnet, dass** das erste Verschlussteil als Rastnase (42) ausgebildet ist, die an einer dem Behälter (12) für Wirkstoff abgewandten Seite (36) der Verdunsteinheit (14) angeordnet ist, und dass das Kappenelement (16) an der Innenseite (44) eine Vertiefung (46) für die Rastnase (42) aufweist, in die die Rastnase (42) in einer vollständig geöffneten Position des Kappenelements (16) einrastet, und dass die Rastnase (42) beim Bewegen zwischen geöffneter und geschlossener Position des Kappenelements (16) die Innenseite (44) des Kappenelements (16) parallel zur Verschiebungsrichtung (52) kontaktiert.

5. Vorrichtung nach Anspruch 4, **dadurch gekennzeichnet, dass** die Verdunsteinheit (14) und das Kappenelement (16) je zwei zur Verschiebungsrichtung (52) im Wesentlichen parallele Seitenflächen (54, 56) aufweisen, dass genau zwei Rastnasen (42) an den Seitenflächen (54) der Verdunsteinheit (14) angeordnet sind und dass genau zwei Vertiefungen (46) an den Seitenflächen (56) des Kappenelements (16) angeordnet sind.

6. Vorrichtung zum Abdunsten von Wirkstoffen, umfassend einen Behälter (12) für den Wirkstoff und eine Verdunsteinheit (14), an der ein Kappenelement (16) angeordnet ist, das relativ zur Verdunsteinheit in eine geöffnete und in eine geschlossene Position verschiebbar ist, wobei die Verdunsteinheit in der geschlossenen Position durch das Kappenelement abgedeckt ist und in der geöffneten Position zumindest teilweise freiliegt, wobei die Vorrichtung eine Verschlussmechanik aufweist, umfassend ein erstes Verschlussteil, das an der Verdunsteinheit (14) angeordnet ist, und ein zweites, zum ersten Verschlussteil komplementäres Verschlussteil, das an einer Innenseite (44) des Kappenelements (16) angeordnet ist, wobei die Verschlussteile derart zusammenwirken, dass sich das erste oder das zweite Verschlussteil bei einer vollständig geöffneten Position des Kappenelements (16) in einer Anschlagposition befindet und beim Bewegen zwischen geöffneter und geschlossener Position des Kappenelements (16) das erste Verschlussteil die Innenseite (44) des Kappenelements (16) oder das zweite Verschlussteil die Verdunsteinheit (14) parallel zur Verschiebungsrichtung (52) kontaktiert, **dadurch gekennzeichnet, dass** das erste Verschlussteil als Vorsprung (64) ausgebildet ist, der an der Verdunsteinheit (14)
beabstandet zum Behälter (12) für Wirkstoff angeordnet ist, dass das Kappenelement (16) an der Innenseite (44) eine als Führungsrille (66) ausgebildete Vertiefung aufweist, in der der Vorsprung (64) beim Bewegen zwischen geöffneter und geschlossener Position des Kappenelements (16) gleitet, und dass an der Innenseite (44) des Kappenelements (16) ein Anschlagelement (65) für den Vorsprung (64) angeordnet ist, an dem der Vorsprung (64) in einer vollständig geöffneten Position des Kappenelements (16) anschlägt.

7. Vorrichtung nach Anspruch 6, **dadurch gekennzeichnet, dass** die Verdunsteinheit (14) und das Kappenelement (16) je zwei zur Verschiebungsrichtung (52) im Wesentlichen parallele Seitenflächen (54, 56) aufweisen, dass genau zwei Vorsprünge (64) an den Seitenflächen (54) der Verdunsteinheit (14) angeordnet sind und dass genau zwei Vertiefungen (66) an den Seitenflächen (56) des Kappenelements (16) angeordnet sind.

8. Vorrichtung nach einem der Ansprüche 1 bis 7, **dadurch gekennzeichnet, dass** außen an dem Kappenelement (16) oder am Behälter (12) für Wirkstoff ein Befestigungselement (28) angeordnet ist.

9. Vorrichtung nach Anspruch 8, **dadurch gekennzeichnet, dass** das Befestigungselement (28) derart ausgeformt ist, dass es zumindest teilweise beabstandet von und parallel zu dem Behälter (12) bzw. von und zum Kappenelement (16) verläuft.

10. Vorrichtung nach Anspruch 9, **dadurch gekennzeichnet, dass** der Behälter (12) bzw. das Kappenelement (16) eine Führungsnut (58) aufweist.

11. Vorrichtung nach Anspruch 9 oder 10, **dadurch gekennzeichnet, dass** der parallel zum Behälter (12) bzw. zum Kappenelement (16) verlaufende Teil (29) des Befestigungselements (28) zumindest teilweise drückbar ist.

12. Vorrichtung nach einem der Ansprüche 9 bis 11, **dadurch gekennzeichnet, dass** das Befestigungselement (28) an einem Ende eine Verdickung (32) aufweist, die den Behälter (12) bzw. das Kappenelement (16) kontaktiert.

13. Vorrichtung nach einem der Ansprüche 1 bis 12, **dadurch gekennzeichnet, dass** das Kappenelement (16) an der dem Behälter (12) abgewandten Seite (18) oder der Behälter (12) an der dem Kappenelement (16) abgewandten Seite (67) abgerundete Ecken (20) aufweist.

14. Vorrichtung nach einem der Ansprüche 1 bis 13, **gekennzeichnet durch** eine Vorderseite (22) und eine Rückseite (24), die im Wesentlichen parallel zueinander verlaufen, wobei die Rückseite (24) in einem Übergangsbereich (26) zur Vorderseite (22) eine abgerundete Form aufweist.

## Claims

1. Device for evaporating active ingredients, comprising a container (12) for the active ingredient and an evaporation unit (14), on which a cap element (16) is arranged, which is displaceable relative to the evaporation unit into an open and into a closed position, the evaporation unit being covered by the cap element in the closed position and being at least partially exposed in the open position, and comprising a closure mechanism comprising a first closure part, which is arranged on the evaporation unit (14), and a second closure part, complementary to the first closure part, which is arranged on an inside (44) of the cap element (16), the closure parts interacting in such a way that the first or second closure part is in a stop position when the cap element (16) is in a fully open position and, when the cap element (16) moves between the open and closed positions, the first closure part is on the inside (44) of the cap element (16) or the second closure part contacts the evaporation unit (14) parallel to the direction of displacement (52), **characterized in that** the first closure part is designed as a latching hook (41) which is arranged on a side (36) of the evaporation unit (14) facing away from the container (12) for active ingredient, that the cap element (16) on the inside (44) has a recess (45) designed as a groove, in which the locking hook (41) slides when moving between the open and closed positions of the cap element (16), and that on the inside (44) of the cap element (16) a stop element (43) for the latching hook (41) is arranged, on which the latching hook (41) abuts in a fully open position of the cap element (16).

2. Device according to claim 1, **characterized in that** the evaporation unit (14) and the cap element (16) each have a side surface that is substantially parallel to the direction of displacement (52), that the locking hook (41) is arranged on the side surface of the evaporation unit (14), and that the recess (45) and the stop element (43) are arranged on the side surface of the cap element (16).

3. Device according to claim 1 or 2, **characterized in that** a guide element (47) is arranged on the evaporation unit (14) on at least one side facing the inside (44) of the cap element (16).

4. Device for evaporating active ingredients, comprising a container (12) for the active ingredient and an evaporation unit (14), on which a cap element (16) is arranged, which is displaceable relative to the evaporation unit into an open and into a closed position, the evaporation unit being covered by the cap element in the closed position and being at least partially exposed in the open position, and comprising a closure mechanism comprising a first closure part, which is arranged on the evaporation unit (14), and a second closure part, complementary to the first closure part, which is arranged on an inside (44) of the cap element (16), the closure parts interacting in such a way that the first or second closure part is in a stop position when the cap element (16) is in a fully open position and, when the cap element (16) moves between the open and closed positions, the first closure part is on the inside (44) of the cap element (16) or the second closure part contacts the evaporation unit (14) parallel to the direction of displacement (52), **characterized in that** the first closure part is designed as a locking lug (42) which is arranged on a side (36) of the evaporation unit (14) facing away from the container (12) for active ingredient, and that the cap element (16) on the inside (44) has a recess (46) for the locking lug (42), into which the locking lug (42) engages in a fully open position of the cap element (16), and that the locking lug (42) when moving between open and closed position of the cap element (16) contacts the inside (44) of the cap element (16) parallel to the direction of displacement (52).

5. Device according to claim 4, **characterized in that** the evaporation unit (14) and the cap element (16) each have two side surfaces (54, 56) which are essentially parallel to the direction of displacement (52), and that exactly two locking lugs (42) on the side surfaces (54 ) of the evaporation unit (14) are arranged and that exactly two recesses (46) are arranged on the side surfaces (56) of the cap element (16).

6. Device for evaporating active ingredients, comprising a container (12) for the active ingredient and an evaporation unit (14), on which a cap element (16) is arranged, which is displaceable relative to the evaporation unit into an open and into a closed position, the evaporation unit being covered by the cap element in the closed position and being at least partially exposed in the open position, and comprising a closure mechanism comprising a first closure part, which is arranged on the evaporation unit (14), and a second closure part, complementary to the first closure part, which is arranged on an inside (44) of the cap element (16), the closure parts interacting in such a way that the first or second closure part is in a stop position when the cap element (16) is in a fully open position and, when the cap element (16) moves between the open and closed positions, the first closure part is on the inside (44) of the cap element (16) or the second closure part contacts the evaporation unit (14) parallel to the direction of displacement (52), **characterized in that** the first closure part is designed as a salient (64) which is arranged on the evaporation unit (14) in a distance to the container (12) for active ingredient, that the cap element (16) on the inside (44) has a recess designed as a guide groove (66), in which salient (64) slides when moving between the open and closed positions of the cap element (16), and that on the inside (44) of the cap element (16 ) a stop element (43) for the salient (64) is arranged, on which the salient (64) abuts in a fully open position of the cap element (16).

7. Device according to claim 6, **characterized in that** the evaporation unit (14) and the cap element (16) each have two side surfaces (54, 56) which are essentially parallel to the direction of displacement (52), and that exactly salients (64) on the side surfaces (54 ) of the evaporation unit (14) are arranged and that exactly two guide grooves (66) are arranged on the side surfaces (56) of the cap element (16).

8. Device according to one of claims 1 to 7, **characterized in that** a fastening element (28) is arranged on the outside of the cap element (16) or of the container (12) for active ingredient.

9. Device according to claim 8, **characterized in that** the fastening element (28) is shaped such that it runs at least partially spaced from and parallel to the container (12) or the cap element (16) respectively.

10. Device according to claim 9, **characterized in that** the container (12) or the cap element (16), respectively, has a guidance groove (58).

11. Device according to claim 9 or 10, **characterized in that** the part (29) of the fastening element (28) running parallel to the container (12) or the cap element (16), respectively, can be at least partially pressed.

12. Device according to one of claims 9 to 11, **characterized in that** the fastening element (28) has a thickening (32) at one end which contacts the container (12) or the cap element (16), respectively,.

13. Device according to one of claims 1 to 12, **characterized in that** the cap element (16) has rounded corners (20) on the side (18) facing away from the container (12) or, respectively, the container (12) has rounded corners (20) on the side (67) facing away from the cap element (16).

14. Device according to one of claims 1 to 3, **characterized by** a front side (22) and a back side (24) which run essentially parallel to one another, the back side (24) having a rounded shape in a transition region (26) to the front side (22).

## Revendications

1. Dispositif de dégagement de substances actives, comprenant un récipient (12) pour la substance active et une unité d'évaporation (14) sur laquelle est disposé un élément de capuchon (16) qui peut être déplacé par rapport à l'unité d'évaporation dans une position ouverte et dans une position fermée, l'unité d'évaporation étant recouverte par le élément de capuchon en position fermée et étant au moins partiellement exposé en position ouverte, le dispositif comprenant un mécanisme de fermeture comprenant une première pièce de fermeture, qui est disposée sur l'unité d'évaporation (14), et une deuxième pièce de fermeture, complémentaire de la première partie de fermeture et étant disposée sur une partie intérieure (44) de l'élément de capuchon (16), les pièces de fermeture interagissantes de telle sorte que la première ou la deuxième pièce de fermeture se trouve dans une position d'arrêt lorsque l'élément de capuchon (16) est dans une position complètement ouverte et que lorsque l'élément de capuchon (16) se déplace entre les positions ouverte et fermée, la première pièce de fermeture se trouve à l'intérieur (44) de l'élément de capuchon (16) ou la deuxième pièce de fermeture entre en contact avec l'unité d'évaporation (14) parallèlement au sens de déplacement (52), **caractérisé en ce que** la première pièce de fermeture est réalisée sous la forme d'un crochet d'encliquetage (41) disposé sur un côté (36) de l'unité d'évaporation (14) opposé au récipient (12) pour substance active, **en ce que** l'élément de capuchon (16) présente à l'intérieur (44) un évidement (45) conçu comme une rainure, dans lequel le crochet d'encliquetage (41) glisse lors du déplacement entre les positions ouverte et fermée de l'élément de capuchon (16), et **en ce qu'**à l'intérieur (44) de l'élément de capuchon (16) est disposé un élément d'arrêt (43) pour le crochet d'encliquetage (41), sur lequel le crochet d'encliquetage (41) vient en butée dans une position complètement ouverte de l'élément de capuchon (16).

2. Dispositif selon la revendication 1, **caractérisé en ce que** l'unité d'évaporation (14) et l'élément de capuchon (16) présentent chacun une surface latérale sensiblement parallèle à la direction de déplacement (52), **en ce que** le crochet d'encliquetage (41) est disposé sur la surface latérale de l'unité d'évaporation (14), et **en ce que** l'évidement (45) et l'élément d'arrêt (43) sont disposés sur la surface latérale de l'élément de capuchon (16).

3. Dispositif selon la revendication 1 ou 2, **caractérisé en ce qu'**un élément de guidage (47) est disposé sur l'unité d'évaporation (14) sur au moins un côté tourné vers l'intérieur (44) de l'élément de capuchon (16).

4. Dispositif de dégagement de substances actives, comprenant un récipient (12) pour la substance active et une unité d'évaporation (14) sur laquelle est disposé un élément de capuchon (16) qui peut être déplacé par rapport à l'unité d'évaporation dans une position ouverte et dans une position fermée, l'unité d'évaporation étant recouverte par le élément de capuchon en position fermée et étant au moins partiellement exposé en position ouverte, le dispositif comprenant un mécanisme de fermeture comprenant une première pièce de fermeture, qui est disposée sur l'unité d'évaporation (14), et une deuxième pièce de fermeture, complémentaire de la première partie de fermeture et étant disposée sur une partie intérieure (44) de l'élément de capuchon (16), les pièces de fermeture interagissantes de telle sorte que la première ou la deuxième pièce de fermeture se trouve dans une position d'arrêt lorsque l'élément de capuchon (16) est dans une position complètement ouverte et que lorsque l'élément de capuchon (16) se déplace entre les positions ouverte et fermée, la première pièce de fermeture se trouve à l'intérieur (44) de l'élément de capuchon (16) ou la deuxième pièce de fermeture entre en contact avec l'unité d'évaporation (14) parallèlement au sens de déplacement (52), **caractérisé en ce que** la première pièce de fermeture est réalisée sous la forme d'un ergot d'encliquetage (42) disposé sur un côté (36) de l'unité d'évaporation (14) opposé au récipient (12) pour substance active, et **en ce que** l'élément de capuchon (16) présente à l'intérieur (44) un évidement (46) pour l'ergot d'encliquetage (42), dans lequel l'ergot d'encliquetage (42) s'enclenche dans une position complètement ouverte de l'élément de capuchon (16), et **en ce que** l'ergot d'encliquetage (42) entre en contact avec l'intérieur (44) de l'élément de capuchon (16) parallèlement à la direction de déplacement (52) lors du déplacement entre la position ouverte et fermée de l'élément de capuchon (16).

5. Dispositif selon la revendication 4, **caractérisé en ce que** l'unité d'évaporation (14) et l'élément de capuchon (16) présentent chacun deux surfaces latérales (54, 56) essentiellement parallèles à la direction de déplacement (52) et **en ce qu'**exactement deux ergots d'encliquetage (42) sont disposées sur les surfaces latérales (54) de l'unité d'évaporation (14) et **en ce que** exactement deux évidements (46) sont disposés sur les surfaces latérales (56) de l'élément de capuchon (16).

6. Dispositif de dégagement de substances actives, comprenant un récipient (12) pour la substance active et une unité d'évaporation (14) sur laquelle est disposé un élément de capuchon (16) qui peut être déplacé par rapport à l'unité d'évaporation dans une position ouverte et dans une position fermée, l'unité d'évaporation étant recouverte par le élément de capuchon en position fermée et étant au moins partiellement exposé en position ouverte, le dispositif comprenant un mécanisme de fermeture comprenant une première pièce de fermeture, qui est disposée sur l'unité d'évaporation (14), et une deuxième pièce de fermeture, complémentaire de la première partie de fermeture et étant disposée sur une partie intérieure (44) de l'élément de capuchon (16), les pièces de fermeture interagissantes de telle sorte que la première ou la deuxième pièce de fermeture se trouve dans une position d'arrêt lorsque l'élément de capuchon (16) est dans une position complètement ouverte et que lorsque l'élément de capuchon (16) se déplace entre les positions ouverte et fermée, la première pièce de fermeture se trouve à l'intérieur (44) de l'élément de capuchon (16) ou la deuxième pièce de fermeture entre en contact avec l'unité d'évaporation (14) parallèlement au sens de déplacement (52), **caractérisé en ce que** la première pièce de fermeture est réalisée sous la forme d'une saillie (64) disposée sur l'unité d'évaporation (14) en distance du récipient (12) pour substance active, et **en ce que** l'élément de capuchon (16) présente à l'intérieur (44) un évidement (46) conformé en rainure de guidage (66), dans lequel la saillie (64) glisse lors du déplacement entre la position ouverte et fermée de l'élément de capuchon (16), et **en ce qu'**à l'intérieur (44) de l'élément de capuchon (16) est disposé un élément d'arrêt (65) pour la saillie (64), sur lequel la saillie (64) vient en butée dans une position complètement ouverte de l'élément de capuchon (16).

7. Dispositif selon la revendication 6, **caractérisé en ce que** l'unité d'évaporation (14) et l'élément de capuchon (16) présentent chacun deux surfaces latérales (54, 56) essentiellement parallèles à la direction de déplacement (52) et **en ce qu'**exactement deux saillies (64) sont disposées sur les surfaces latérales (54) de l'unité d'évaporation (14) et **en ce que** exactement deux évidements (66) sont disposés sur les surfaces latérales (56) de l'élément de capuchon (16).

8. Dispositif selon l'une des revendications 1 à 7, **caractérisé en ce qu'**un élément de fixation (28) est disposé à l'extérieur de l'élément de capuchon (16) ou du récipient (12) pour substance active.

9. Dispositif selon la revendication 8, **caractérisé en ce que** l'élément de fixation (28) est conformé de telle sorte qu'il s'étend au moins partiellement à distance et parallèlement au récipient (12) ou bien à l'élément de capuchon (16).

10. Dispositif selon la revendication 9, **caractérisé en ce que** le récipient (12) ou bien l'élément de capuchon (16) présente une rainure de guidage (58).

11. Dispositif selon la revendication 9 ou 10, **caractérisé en ce que** la partie (29) de l'élément de fixation (28) s'étendant parallèlement au récipient (12) ou bien à l'élément de capuchon (16) peut être pressée au moins partiellement.

12. Dispositif selon l'une des revendications 9 à 11, **caractérisé en ce que** l'élément de fixation (28) présente à une extrémité un épaississement (32) en contact avec le récipient (12) ou bien à l'élément de capuchon (16).

13. Dispositif selon l'une des revendications 1 à 12, **caractérisé en ce que** ou l'élément de capuchon (16) présente des coins arrondis (20) sur le côté (18) opposé au récipient (12) ou bien le récipient (12) présente des coins arrondis (20) sur le côté (67) opposé à l'élement de capuchon (16).

14. Dispositif selon l'une des revendications 1 à 3, **caractérisé par** une face avant (22) et une face arrière (24) qui s'étendent essentiellement parallèlement l'une à l'autre, la face arrière (24) présentant une forme arrondie dans une zone de transition (26) vers la face avant (22).
